# EUROPEAN PATENT APPLICATION

(11) **EP 4 223 779 A1**
(43) Date of publication of application: **09.08.2023**
(21) Application number: 21874525.5
(22) Date of filing: 29.09.2021
(51) Int. Cl.: C07K 16/28, C07K 19/00, A61K 39/00, A61K 39/395, C12N 15/62, C12N 5/10, A61P 35/00

(54) **EGFR-TARGETING CHIMERIC ANTIGEN RECEPTOR**

(30) Priority: 30.09.2020 CN 202011062635
(71) Applicant: Institute Of Zoology, Chinese Academy Of Sciences, Beijing 100101 (CN); Beijing Institute for Stem Cell and Regenerative Medicine, Beijing 100101 (CN)
(72) Inventor: WANG, Haoyi, Beijing 100101 (CN); XU, Beilei, Beijing 100101 (CN); LI, Na, Beijing 100101 (CN); TANG, Na, Beijing 100101 (CN)
(74) Representative: Michalski Hüttermann & Partner Patentanwälte mbB
(86) International application number: PCT/CN2021/121678
(87) International publication number: WO 2022/068870

(57) **Abstract**

The present invention relates to the field of biomedicines. Specifically, the present invention relates to a chimeric antigen receptor (CAR) targeting EGFR, a CAR-T cell containing the CAR, as well as a preparation method and use thereof.

## Description

### Technical Field

The present invention relates to the field of biomedicines. Specifically, the present invention relates to a chimeric antigen receptor (CAR) targeting EGFR, a CAR-T cell containing the CAR, as well as a preparation method and use thereof.

### Background Art

Human epidermal growth factor receptor (also known as HER-1 or Erb-B1, and referred to as "EGFR" herein) is a 170 kDa transmembrane receptor encoded by a c-erbB proto-oncogene, and shows the inherent tyrosine kinase activity. EGFR regulates various cellular processes by signal transduction pathways mediated by a tyrosine kinase, including but not limited to the activation of the signal transduction pathways that control cell proliferation, differentiation, cell survival, apoptosis, angiogenesis, mitogenesis and metastasis.

It is indicated from researches that the increase or overexpression of the EGFR gene copy number may promote the malignant transformation of normal cells and the metastasis of malignant tumors, and the signal transduction network of EGFR plays an important role in formation and development processes of tumors. The overexpression of EGFR is already reported in researches of many human malignant tumors, including lung cancer, pancreatic cancer, colorectal cancer, gastric cancer and breast cancer and the like. In addition, it is indicated from clinical research results that the overexpression of EGFR is associated with poor prognosis in patients. EGFR already becomes a specific target for anti-tumor therapy.

Some drugs targeting EGFR are already approved for clinical treatment of the human malignant tumors. These drugs are mainly divided into two categories: the first category is monoclonal antibody drugs that block an extracellular functional region of EGFR, such as Cetuximab, Panitumumab and Nimotuzumab; and the other category is small-molecule tyrosine kinase inhibitors targeting an intracellular region of EGFR, such as Gefitinib, Erlotinib and Afatinib. Although the safety and clinical efficacy of these drugs are already proven, in many cases, their anti-tumor effects are not as effective as expected, there are problems such as a decrease in blood concentration of monoclonal antibody targeted drugs over time, a low target response rate, and EGFR mutations.

Therefore, new drugs and therapies for treating EGFR related malignant tumors are still needed in this field.

### Summary of the Invention

In one aspect, the present invention provides a chimeric antigen receptor (CAR) targeting EGFR, which comprises an extracellular antigen binding domain specifically targeting EGFR, wherein the extracellular antigen binding domain comprises a heavy chain variable region (VH) and a light chain variable region (VL), wherein
i) the VH comprises VH-CDR1 shown in SEQ ID NO: 1, VH-CDR2 shown in SEQ ID NO: 2, and VH-CDR3 shown in SEQ ID NO: 3, and the VL comprises VL-CDR1 shown in SEQ ID NO: 4, VL-CDR2 shown in SEQ ID NO: 5, and VL-CDR3 shown in SEQ ID NO: 6;
ii) the VH comprises VH-CDR1 shown in SEQ ID NO: 10, VH-CDR2 shown in SEQ ID NO: 11, and VH-CDR3 shown in SEQ ID NO: 12, and the VL comprises VL-CDR1 shown in SEQ ID NO: 13, VL-CDR2 shown in SEQ ID NO: 14, and VL-CDR3 shown in SEQ ID NO: 16;
iii) the VH comprises VH-CDR1 shown in SEQ ID NO: 19, VH-CDR2 shown in SEQ ID NO: 20, and VH-CDR3 shown in SEQ ID NO: 21, and the VL comprises VL-CDR1 shown in SEQ ID NO: 22, VL-CDR2 shown in SEQ ID NO: 23, and VL-CDR3 shown in SEQ ID NO: 24;
iv) the VH comprises VH-CDR1 shown in SEQ ID NO: 28, VH-CDR2 shown in SEQ ID NO: 29, and VH-CDR3 shown in SEQ ID NO: 30, and the VL comprises VL-CDR1 shown in SEQ ID NO: 31, VL-CDR2 shown in SEQ ID NO: 32, and VL-CDR3 shown in SEQ ID NO: 33;
v) the VH comprises VH-CDR1 shown in SEQ ID NO: 37, VH-CDR2 shown in SEQ ID NO: 38, and VH-CDR3 shown in SEQ ID NO: 39, and the VL comprises VL-CDR1 shown in SEQ ID NO: 40, VL-CDR2 shown in SEQ ID NO: 41, and VL-CDR3 shown in SEQ ID NO: 42; or
vi) the VH comprises VH-CDR1 shown in SEQ ID NO: 46, VH-CDR2 shown in SEQ ID NO: 47, and VH-CDR3 shown in SEQ ID NO: 48, and the VL comprises VL-CDR1 shown in SEQ ID NO: 49, VL-CDR2 shown in SEQ ID NO: 50, and VL-CDR3 shown in SEQ ID NO: 51.

In some embodiments, wherein
i) the VH comprises the amino acid sequence shown in SEQ ID NO: 7, and the VL comprises the amino acid sequence shown in SEQ ID NO: 8;
ii) the VH comprises the amino acid sequence shown in SEQ ID NO: 16, and the VL comprises the amino acid sequence shown in SEQ ID NO: 17;
iii) the VH comprises the amino acid sequence shown in SEQ ID NO: 25, and the VL comprises the amino acid sequence shown in SEQ ID NO: 26;
iv) the VH comprises the amino acid sequence shown in SEQ ID NO: 34, and the VL comprises the amino acid sequence shown in SEQ ID NO: 35;
v) the VH comprises the amino acid sequence shown in SEQ ID NO: 43, and the VL comprises the amino acid sequence shown in SEQ ID NO: 44; or
vi) the VH comprises the amino acid sequence shown in SEQ ID NO: 52, and the VL comprises the amino acid sequence shown in SEQ ID NO: 53.

In some embodiments, wherein the extracellular antigen binding domain includes a single stranded Fv fragment (scFv).

In some embodiments, wherein the scFv comprises an amino acid sequence selected from SEQ ID NOs: 9, 18, 27, 36, 45 and 54.

In some embodiments, wherein the CAR further comprises a CD8α signal peptide at N-terminus, for example, the CD8α signal peptide comprises the amino acid sequence of SEQ ID NO: 55.

In some embodiments, wherein the CAR further comprises a transmembrane domain, such as a CD8α transmembrane domain, for example, the CD8α transmembrane domain comprises the amino acid sequence of SEQ ID NO: 57.

In some embodiments, the CAR further comprises a hinge region located between the extracellular antigen binding domain and the transmembrane domain, for example, the hinge region is a CD8α hinge region, for example, the CD8α hinge region comprises the amino acid sequence of SEQ ID NO: 56.

In some embodiments, the CAR further comprises a signal transduction domain, such as a CD3ζ signal transduction domain, for example, the CD3ζ signal transduction domain comprises the amino acid sequence shown in SEQ ID NO: 59.

In some embodiments, the CAR further comprises one or more co-stimulatory domains, such as a 4-1BB co-stimulatory domain, for example, the 4-1BB co-stimulatory domain comprises the amino acid sequence of SEQ ID NO: 58.

In some embodiments, the CAR comprises an amino acid sequence selected from SEQ ID NOs: 60-65.

In one aspect, the present invention provides a therapeutic T cell, which comprises CAR of the present invention.

In some embodiments, a TGFβ receptor (such as TGFBRI, TGFBRII, and TGFBRIII) in the therapeutic T cell is knocked down or knocked out.

In some embodiments, the therapeutic T cell can specifically lyse tumor cells expressing EGFR in vitro at an effect-target ratio of about 0.2:1 to about 0.00625:1, for example, about 0.2:1, about 0.1:1, about 0.05:1, about 0.025:1, about 0.0125:1, and about 0.00625:1.

In one aspect, the present invention provides a use of the therapeutic T cell of the present invention in preparation of a drug for treating an EGFR related cancer.

In one aspect, the present invention provides a pharmaceutical composition for treating an EGFR related cancer in a subject, which comprises a therapeutically effective amount of the therapeutic T cells of the present invention and a pharmaceutically acceptable carrier.

In one aspect, the present invention provides a method for treating an EGFR related cancer, including administering a therapeutically effective amount of the therapeutic T cells of the present invention or the pharmaceutical composition of the present invention to a subject in need.

In some embodiments, the method further includes administering radiation therapy and/or chemotherapy and/or another tumor targeted drug and/or immunotherapy to the subject.

In some embodiments of each aspect of the present invention, the EGFR related cancer is selected from esophageal cancer, gastric cancer, colon cancer, rectal cancer, colorectal cancer, pancreatic cancer, lung cancer (including non-small cell lung cancer NSCLC), breast cancer, cervical cancer, corpus cancer, endometrial cancer, ovarian cancer, bladder cancer, head and neck cancer (including head and neck squamous cell cancer SCCHN), osteosarcoma, prostate cancer, neuroblastoma, renal cancer, glioma, glioblastoma and skin cancer (including epithelial cancer).

In one aspect, the present invention provides a polynucleotide, which comprises a nucleotide sequence encoding the CAR of the present invention. In some embodiments, the polynucleotide comprises a nucleotide sequence selected from SEQ ID NOs: 66-71.

In one aspect, the present invention provides an expression construct, which comprises the polynucleotide of the present invention operably linked to a regulatory sequence.

In one aspect, the present invention provides a method for preparing the therapeutic T cell of the present invention, and the method includes the following steps:
a) providing a isolated T cell; and
b) introducing the polynucleotide of the present invention or the expression construct of the present invention into the T cell, thereby causing the T cell to express the CAR of the present invention.

In some embodiments, the method further includes a step:
x) knocking down or knocking out the TGFβ receptor (such as TGFBRI, TGFBRII, and TGFBRIII) in the T cell.

### Brief Description of the Drawings

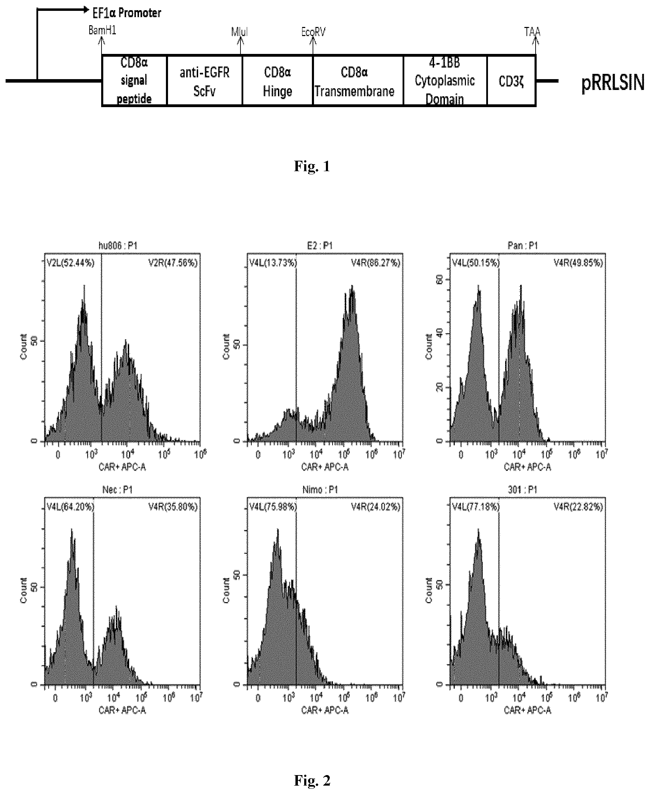

Fig. 1 is a schematic diagram of a lentivirus vector target gene sequence of anti-EGFR CAR.
Fig. 2 is detection of positive rates of 6 anti-EGFR CAR-T cells.

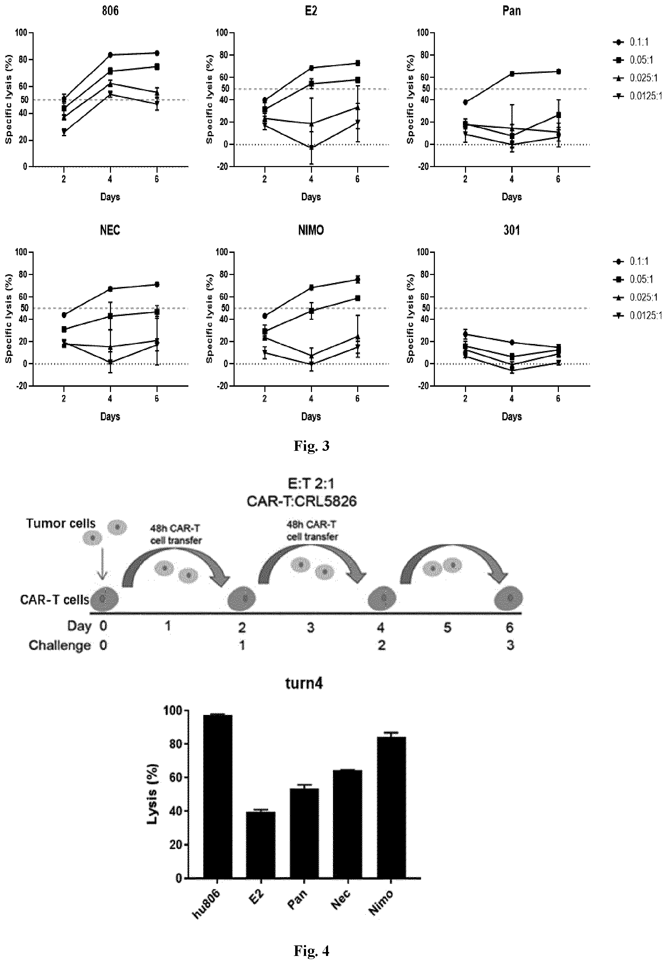

Fig. 3 shows that 6 humanized anti-EGFR CAR-T cells are co-cultured with CRL-5826 cells for a long term in vitro, and the Luciferase content in the CRL-5826 cells is measured to quantify the tumor killing value (N=4, SEM).
Fig. 4 shows comparison of in vitro functions of 6 humanized anti-EGFR CAR-T cells: in vitro Stress Test experiment of 6 humanized anti-EGFR CAR-T cells. Every other day, the CAR-T cells were taken from a killing sample well, new tumor target cells were added in the ratio of E:T=2:1, and the killing rate eas detected (target cell: CRL-5826, E:T=2:1, 50% CAR-T positive rate, N=4, SEM).

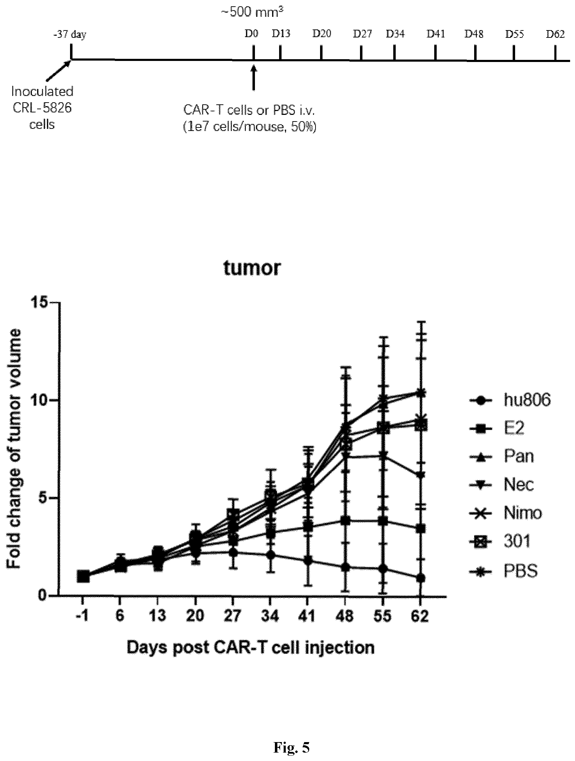

Fig. 5 shows comparison of in vivo functions of 6 humanized anti-EGFR CAR-T cells. A) NPG mouse experimental process. Tumor cell inoculation amount: 2×10⁶/mouse, CAR-T cell injection dose: 1×10⁷/mouse, 50% CAR positive, i.v.: tail vein injection, with five NPG mice in each group. B) Changes of tumor volumes in mice (N=5, SEM).

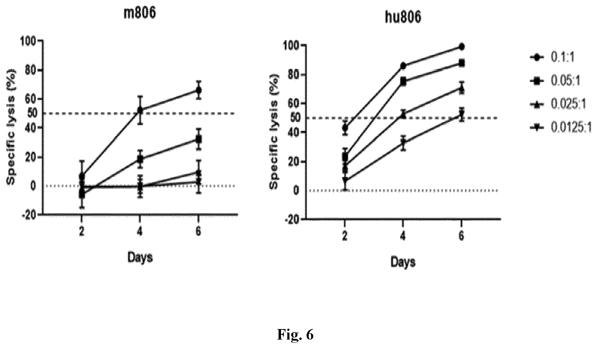

Fig. 6 shows comparison of in vitro functions between humanized hu806 CAR-T cells and mouse-derived m806 CAR-T cells. CAR-T cells were co-cultured with the CRL-5826 cells in vitro for a long term, and the Luciferase content in the CRL-5826 cells was detected to quantify the tumor killing value (N=4, SEM).

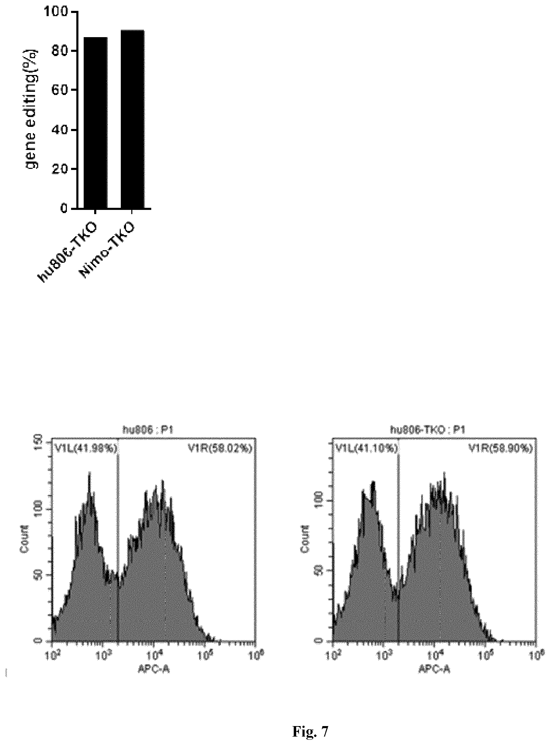

Fig. 7 shows the hu806 CAR-T cells with TGF-β Receptor II knocked out. A) The knockout efficiency of hu806-TKO CAR-T cells was detected by TIDE method. B) The positive rates of hu806 CAR-T and hu806-TKO CAR-T cells were detected by flow cytometry.

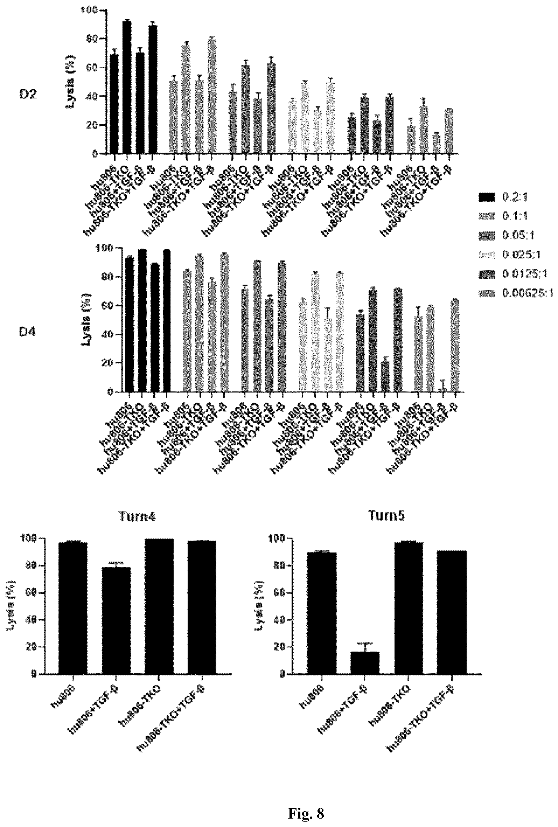

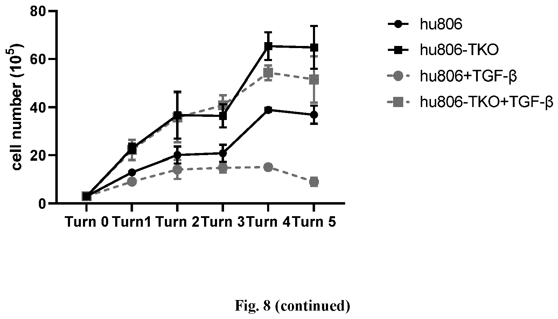

Fig. 8 shows detection of killing functions of the hu806 CAR-T cells with TGF-β Receptor II knocked out. A) Detection of long-term killing of the CRL-5826 cells in vitro. TGF-β final concentration: 5 ng/µl, (N=4, SEM). B. Detection of the fourth and fifth round killing of the hu806 CAR-T and hu806-TKO CAR-T cells. Added TGF-β final concentration: 5 ng/µl. (N=4, SEM). C) Cell proliferation statistics of the hu806 and hu806-TKO CAR-T cells in the Stress-Test experiment.

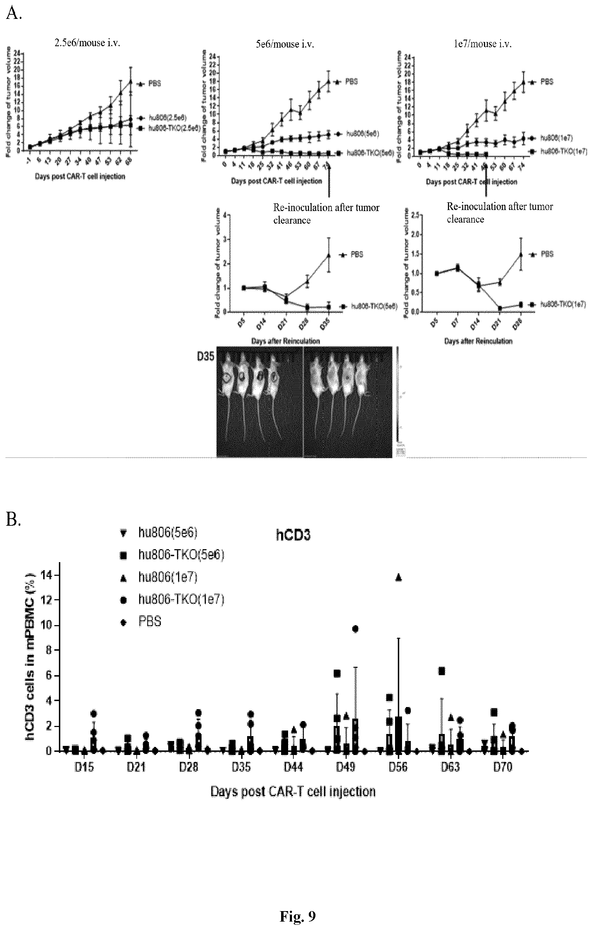

Fig. 9 shows the mouse in vivo experiment. A) Different doses of CAR-T cells were injected into tail veins of the NPG mice, resulting in changes in tumor sizes, and changes in tumor sizes after tumor re-inoculation. Five mice in each group were injected with CAR-T, and the positive rate was 50%. B) Human CD3 content in peripheral blood of the mice in each experimental group and PBS group was detected by the flow cytometry.

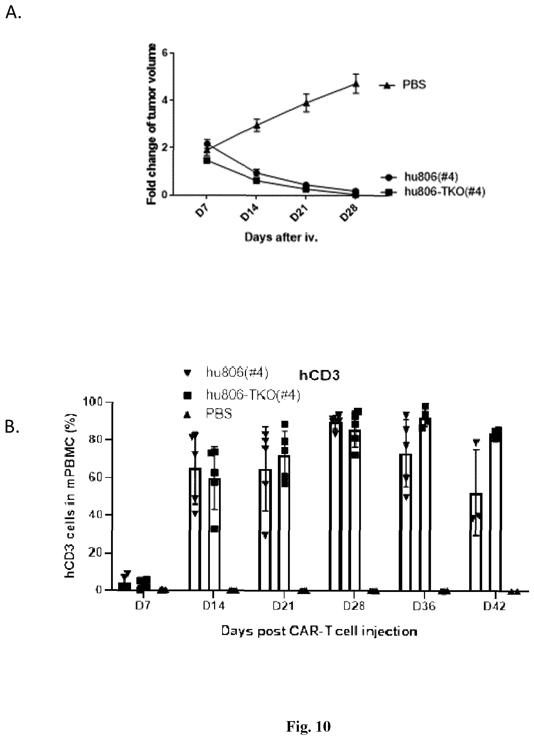

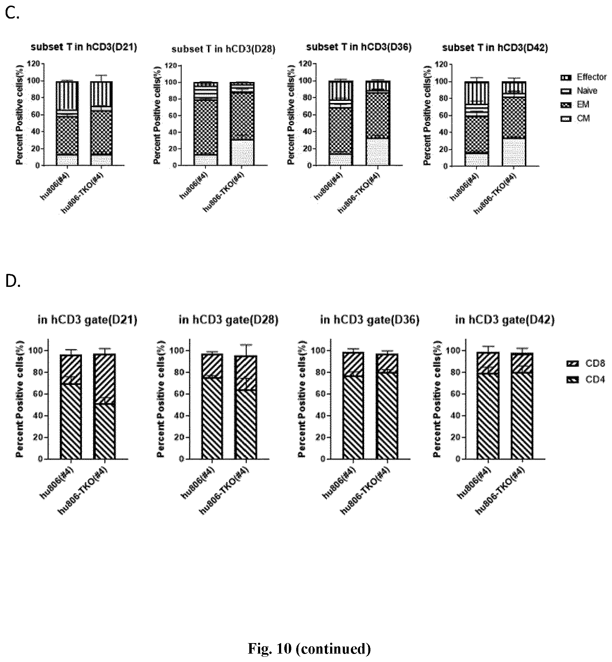

Fig. 10 shows an analysis experiment of various subtypes of huCD3 in the mice. A) After the CAR-T cells (1×10⁷ cells/mouse, 50% CAR positive) were injected to the tail veins of the NPG mice, the tumor size was changed. CAR-TT cells were prepared by using #4 donor peripheral blood with strong in vivo amplification ability. There were five mice in each group. B) The human CD3 content in the peripheral blood of the mice in each experimental group and PBS group was detected by flow cytometry. C) The proportion of T cell subtypes in the peripheral blood of the mice was detected by the flow cytometry. D) The proportion of huCD4 and huCD8 to huCD3 in the peripheral blood of the mice was detected by the flow cytometry. The peripheral blood was collected from two groups of the mice on Day 21, 28, 36 and 42 for analysis.

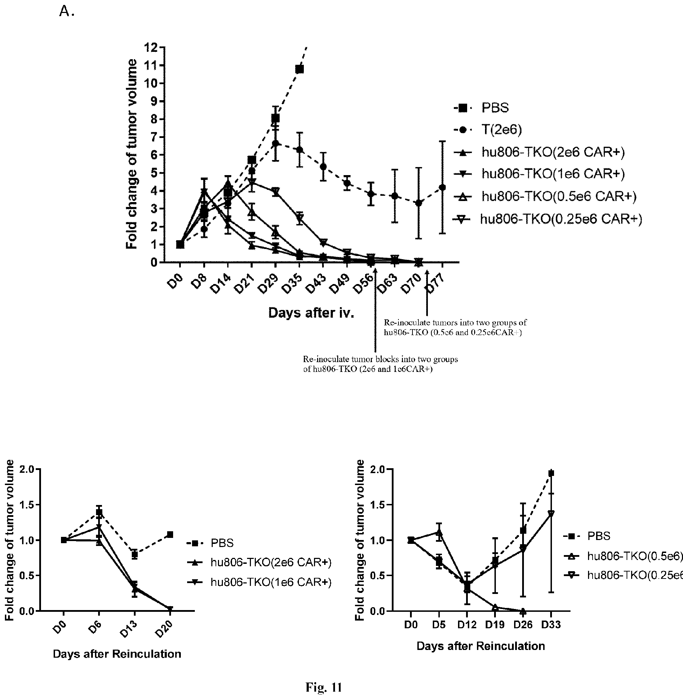

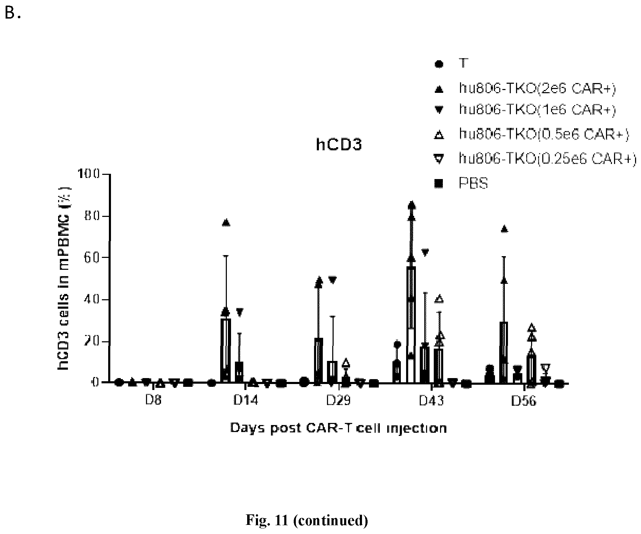

Fig. 11 shows in vivo therapeutic dose experiment of the Hu806-TKO CAR-T cells on tumor-bearing NPG mice. A) Different doses of CAR-T cells (respectively: 2×10⁶ CAR+ cells/mouse, 1×10⁶ CAR+ cells/mouse, 0.5×10⁶ CAR+ cells/mouse and 0.25×10⁶ CAR+ cells/mouse) were injected into tail veins of the NPG mice, the tumor size changed. After the tumor was completely cleared, it was re-inoculated and the tumor size changed. There were 5 mice in each group. B) The human CD3 content in the peripheral blood of the mice in each experimental group and control group was detected by the flow cytometry.

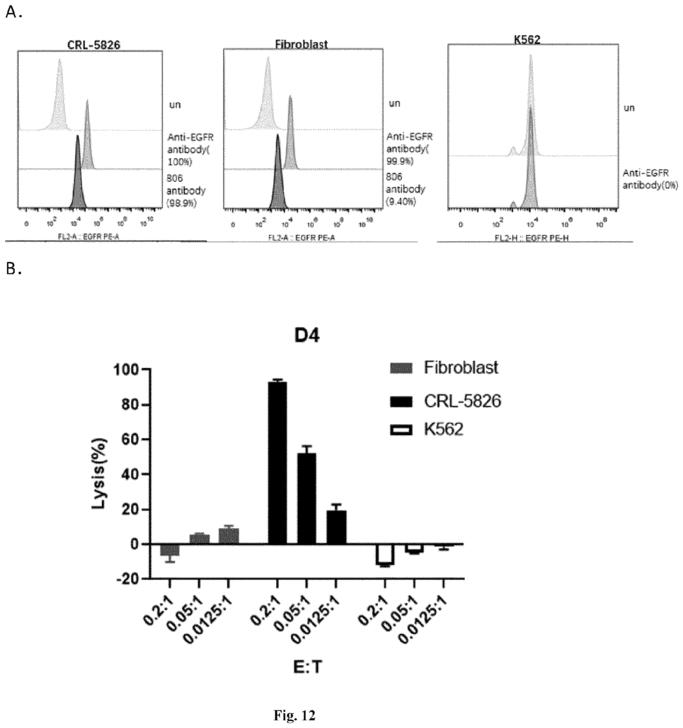

Fig. 12 shows off-target safety detection of the Hu806 CAR-T cells. A) Flow cytometry detection of lung squamous cell carcinoma cell line CRL-5826, human dermal fibroblast Fibroblast, and leukemia cell line K562. Staining antibody: anti-EGFR antibody-PE and 806 antibody-PE. B) Detection of in vitro killing functions of the Hu806 CAR-T cells against three types of cells. A real-time unlabeled cell analysis technology detection method (N=2, SEM) was adopted.

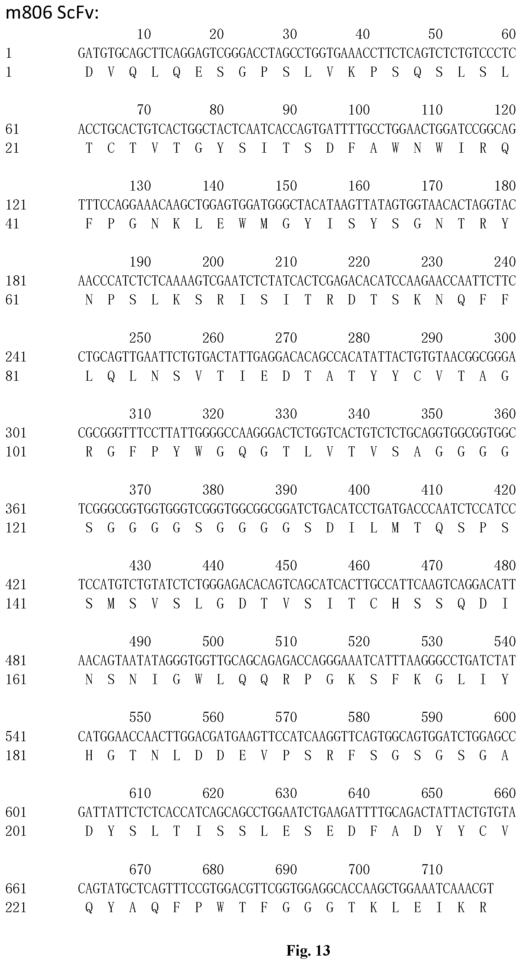

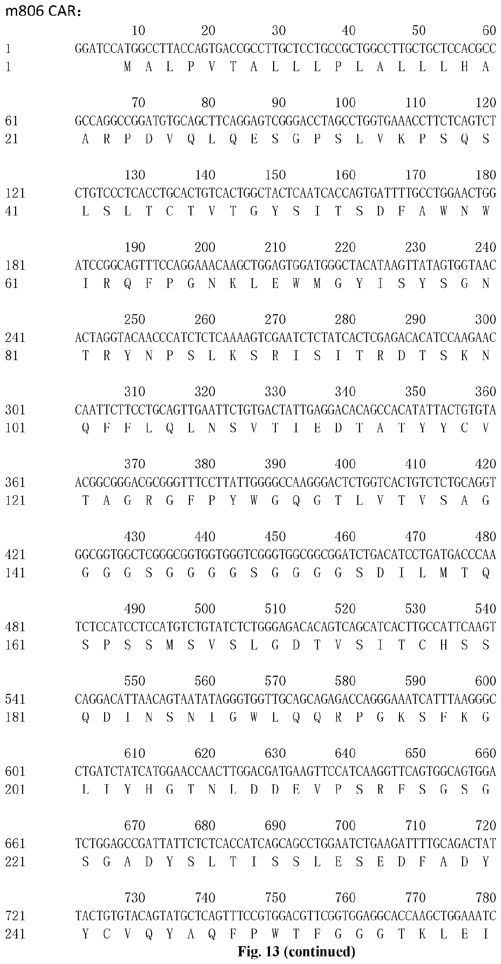

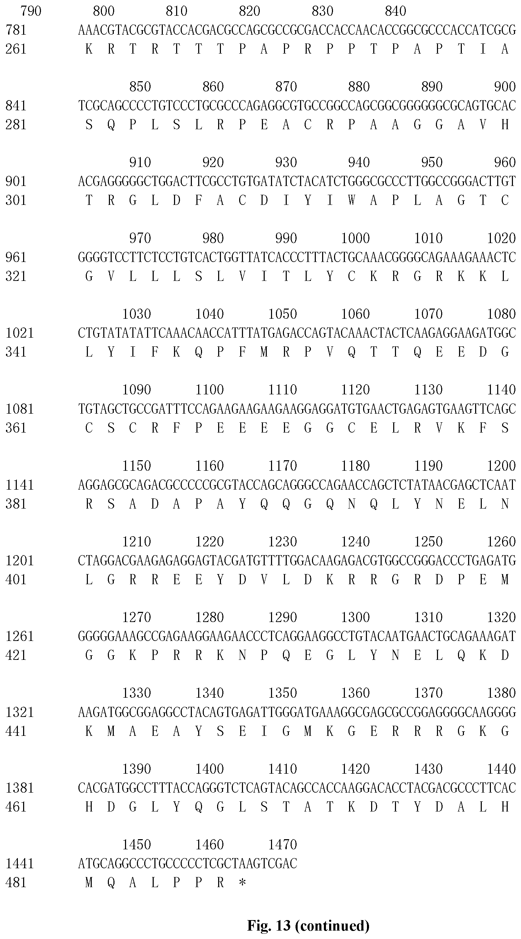

Fig. 13 is a corresponding relationship diagram of amino acid sequences and nucleotide sequences of m806 scFv and CAR.

### Detailed Description of the Invention

Unless otherwise indicated or defined, all terms used have ordinary meanings in the art, the meanings may be understood by those skilled in the art. Reference can be made to standard manuals such as Sambrook et al., "Molecular Cloning: A Laboratory Manual"; Lewin, "Genes VIII"; and Roitt et al., "Immunology" (the 8-th edition), as well as general existing technologies cited herein; in addition, unless otherwise specified, all methods, steps, technologies and operations that are not specifically detailed may be and already have been performed in a known manner, which may be understood by those skilled in the art. Reference can also be made to the standard manuals, the above general existing technologies, and other reference documents cited therein.

As used herein, the term "and/or" covers all combinations of items connected by this term, and should be considered that each combination is already separately listed herein. For example, "A and/or B" encompasses "A", "A and B", and "B". For example, "A, B and/or C" encompasses "A", "B", "C", "A and B", "A and C", "B and C", and "A and B and C".

When the term "comprises/comprising" is used herein to describe a sequence of a protein or nucleic acid, the protein or nucleic acid may be composed of the sequence, or may have additional amino acids or nucleotides at one or both terminus of the protein or nucleic acid, but still possess the activity described in the present invention. In addition, those skilled in the art are aware that methionine encoded by a starting codon at the N-terminus of the peptide may be retained in some practical situations (such as when expressed in specific expression systems), but it does not substantially affect the functionality of the peptide. Therefore, when the specific amino acid sequence is described in the description and claims of the present application, although it may not contain the N-terminus methionine encoded by the starting codon, it also encompasses a sequence containing the methionine. Correspondingly, a nucleotide sequence encoded by it may also contain the starting codon; and vice versa.

In a first aspect, the present invention provides a chimeric antigen receptor (CAR) targeting EGFR, which comprises an extracellular antigen binding domain specifically targeting EGFR, the extracellular antigen binding domain comprises a heavy chain variable region (VH) and a light chain variable region (VL), wherein
i) the VH comprises VH-CDR1 shown in SEQ ID NO: 1, VH-CDR2 shown in SEQ ID NO: 2, and VH-CDR3 shown in SEQ ID NO: 3, and the VL comprises VL-CDR1 shown in SEQ ID NO: 4, VL-CDR2 shown in SEQ ID NO: 5, and VL-CDR3 shown in SEQ ID NO: 6;
ii) the VH comprises VH-CDR1 shown in SEQ ID NO: 10, VH-CDR2 shown in SEQ ID NO: 11, and VH-CDR3 shown in SEQ ID NO: 12, and the VL comprises VL-CDR1 shown in SEQ ID NO: 13, VL-CDR2 shown in SEQ ID NO: 14, and VL-CDR3 shown in SEQ ID NO: 16;
iii) the VH comprises VH-CDR1 shown in SEQ ID NO: 19, VH-CDR2 shown in SEQ ID NO: 20, and VH-CDR3 shown in SEQ ID NO: 21, and the VL comprises VL-CDR1 shown in SEQ ID NO: 22, VL-CDR2 shown in SEQ ID NO: 23, and VL-CDR3 shown in SEQ ID NO: 24;
iv) the VH comprises VH-CDR1 shown in SEQ ID NO: 28, VH-CDR2 shown in SEQ ID NO: 29, and VH-CDR3 shown in SEQ ID NO: 30, and the VL comprises VL-CDR1 shown in SEQ ID NO: 31, VL-CDR2 shown in SEQ ID NO: 32, and VL-CDR3 shown in SEQ ID NO: 33;
v) the VH comprises VH-CDR1 shown in SEQ ID NO: 37, VH-CDR2 shown in SEQ ID NO: 38, and VH-CDR3 shown in SEQ ID NO: 39, and the VL comprises VL-CDR1 shown in SEQ ID NO: 40, VL-CDR2 shown in SEQ ID NO: 41, and VL-CDR3 shown in SEQ ID NO: 42; or
vi) the VH comprises VH-CDR1 shown in SEQ ID NO: 46, VH-CDR2 shown in SEQ ID NO: 47, and VH-CDR3 shown in SEQ ID NO: 48, and the VL comprises VL-CDR1 shown in SEQ ID NO: 49, VL-CDR2 shown in SEQ ID NO: 50, and VL-CDR3 shown in SEQ ID NO: 51.

In some embodiments, the extracellular antigen binding domain comprises a VH and a VL, wherein
i) the VH comprises the amino acid sequence shown in SEQ ID NO: 7, and the VL comprises the amino acid sequence shown in SEQ ID NO: 8;
ii) the VH comprises the amino acid sequence shown in SEQ ID NO: 16, and the VL comprises the amino acid sequence shown in SEQ ID NO: 17;
iii) the VH comprises the amino acid sequence shown in SEQ ID NO: 25, and the VL comprises the amino acid sequence shown in SEQ ID NO: 26;
iv) the VH comprises the amino acid sequence shown in SEQ ID NO: 34, and the VL comprises the amino acid sequence shown in SEQ ID NO: 35;
v) the VH comprises the amino acid sequence shown in SEQ ID NO: 43, and the VL comprises the amino acid sequence shown in SEQ ID NO: 44; or
vi) the VH comprises the amino acid sequence shown in SEQ ID NO: 52, and the VL comprises the amino acid sequence shown in SEQ ID NO: 53.

In some embodiments, the extracellular antigen binding domain includes a single stranded Fv fragment (scFv).

In some embodiments, the VH and VL are linked by a linker. In some embodiments, the linker is a flexible peptide linker. In some embodiments, the linker comprises the amino acid sequence shown in SEQ ID NO: 72.

In some embodiments, the scFv comprises an amino acid sequence selected from SEQ ID NOs: 9, 18, 27, 36, 45 and 54.

In some embodiments, the CAR further comprises a CD8α signal peptide at the N-terminus. In some embodiments, the CD8α signal peptide comprises the amino acid sequence of SEQ ID NO: 55.

In some embodiments, the CAR further comprises a transmembrane domain, such as a CD8α transmembrane domain or a CD28 transmembrane domain. In some embodiments, the CAR comprises a CD8α transmembrane region. In some embodiments, the CD8α transmembrane region comprises the amino acid sequence of SEQ ID NO: 57.

In some embodiments, the CAR further includes a hinge region located between the extracellular antigen binding domain and the transmembrane domain, for example, the hinge region is a CD8α hinge region. In some embodiments, the CD8α hinge region comprises the amino acid sequence of SEQ ID NO: 56.

In some implementation modes, the CAR further comprises a signal transduction domain, such as a signal transduction domain that may be used for T cell activation, and such as a signal transduction domain selected from TCRζ, FcRγ, FcRβ, FcRε, CD3γ, CD3δ, CD3ε, CD3ζ, CD5, CD22, CD79a, CD79b and CD66d. In some preferred embodiments, the CAR comprises a CD3ζ signal transduction domain, for example, the CD3ζ signal transduction domain comprises the amino acid sequence shown in SEQ ID NO: 59.

In some implementation modes, the CAR further comprises one or more co-stimulatory domains, such as a co-stimulatory domain selected from CD3, CD27, CD28, CD83, CD86, CD127, 4-1BB, and 4-1BBL. In some embodiments, the CAR further comprises a 4-1BB co-stimulatory domain. In some embodiments, the 4-1BB co-stimulatory domain comprises the amino acid sequence of SEQ ID NO: 58.

In some implementation modes, the CAR comprises an extracellular antigen binding domain, a CD8α hinge region, a CD8α transmembrane region, a 4-1BB co-stimulatory domain, and a CD3ζ signal transduction domain targeting EGFR, and optional a CD8α signal peptide at the N-terminus.

In some specific embodiments, the CAR comprises an amino acid sequence selected from SEQ ID NOs: 60-65.

In another aspect, the present invention provides a polynucleotide, which comprises a nucleotide sequence encoding the CAR of the present invention. In some embodiments, the polynucleotide comprises a nucleotide sequence selected from SEQ ID NOs: 66-71.

In another aspect, the present invention provides an expression construct, which comprises the polynucleotide of the present invention operably linked to a regulatory sequence.

The "expression construct" of the present invention may be a linear nucleic acid fragment, a circular plasmid, a viral vector, or a translatable RNA (such as mRNA). In some preferred embodiments, the expression construct is a viral vector, such as a lentivirus vector.

The term "regulatory sequence" and "regulatory element" may be interchangeably used to refer to a nucleotide sequence located at the upstream (5' non-encoding sequence), middle or downstream (3' non-encoding sequence) of an encoding sequence and affecting the transcription, RNA processing or stability or translation of the related encoding sequence. The expression regulatory element refers to a nucleotide sequence that may control the transcription, RNA processing or stability or translation of the interested nucleotide sequence. The regulatory sequence may include but not limited to a promoter, a translation leader sequence, an intron, an enhancer and a polyadenylation recognition sequence.

As used herein, the term "operably linked" refers to the linkage between the regulatory element (such as but not limited to a promoter sequence, and a transcription termination sequence) and the nucleic acid sequence (such as an encoding sequence or an open reading frame), so that the transcription of the nucleotide sequence is controlled and regulated by the transcription regulatory element. The technology for operably linking a regulatory element region to a nucleic acid molecule is known in the art.

In another aspect, the present invention provides a therapeutic T cell, which comprises the CAR of the present invention. In some embodiments, the CAR is expressed on the cell membrane surface of the T cell.

In some embodiments, a TGFβ receptor (such as TGFBRI, TGFBRII, and TGFBRIII) of the therapeutic T cell is knocked down or knocked out.

As used herein, the "TGFβ receptor (such as TGFBRI, TGFBRII, and TGFBRIII) of the therapeutic T cell is knocked down or knocked out" means that relative to a control T cell, the expression of the TGFβ receptor (such as TGFBRI, TGFBRII, and TGFBRIII) in the therapeutic T cell of the present invention is down-regulated or unexpressed, or the activity of the TGFβ receptor (such as TGFBRI, TGFBRII, and TGFBRIII) is reduced or inactivated (such as antagonized). The knockdown or knockout used herein may be at a genome level, a transcription level, a translation level, or a post-translation level.

In some embodiments of each aspect of the present invention, the therapeutic T cell is derived from an autologous cell of a subject. As used herein, the "autologous" refers to that the cell, the cell line, or the cell population used for treatment of a subject is derived from the subject. In some embodiments, the therapeutic T cell is derived from allogeneic cells, for example, derived from a donor subject who is human leukocyte antigen (HLA) compatible with the subject to be treated. A standard scheme may be used to convert a cell from the donor subject into a non-allogeneic reactive cell which can be replicated as needed to generate cells that may be applied to one or more subjects.

In some embodiments, the T cell is derived from a healthy subject. In some embodiments, the T cell is derived from a subject suffering from cancer.

The T cell described in the context of the present invention may be derived from an inflammatory T lymphocyte, a cytotoxic T lymphocyte, a regulatory T lymphocyte, and/or a helper T lymphocyte. In some embodiments, the T cell described in the context of the present invention may be derived from a CD4+T lymphocyte and/or a CD8+T lymphocyte.

The T cell in the context of the present invention may be obtained from many non-limiting sources by various non-limiting methods, including peripheral blood monocyte, bone marrow, lymph node tissue, umbilical cord blood, thymic tissue, ascites, pleural effusion, spleen tissue, and tumor. The T cell in the context of the present invention may also be a part of a mixed population of cells presenting different phenotypic characteristics.

The therapeutic T cell of the present invention may specifically lyse a tumor cell expressing EGFR in vitro. For example, by co-culturing with the tumor cells expressing EGFR in vitro, the therapeutic T cell of the present invention may effectively and specifically lyse the tumor cells expressing EGFR at an effect-target ratio of about 0.2:1 to about 0.00625:1, such as about 0.2:1, about 0.1:1, about 0.05:1, about 0.025:1, about 0.0125:1, and about 0.00625:1 (therapeutic T cell: tumor cell expressing EGFR). For example, after co-culturing for 1, 2, 3, 4, 5, 6, or 7 days, at least about 10%, at least about 20%, at least about 30%, at least about 40%, at least about 50%, at least about 60%, at least about 70%, at least about 80%, and even at least about 90% or more tumor cells expressing EGFR are lysed.

As used in the context of the present application, the "subject" refers to an organism that is suffered from or susceptible to a disease that can be treated by the cell, pharmaceutical composition, or method of the present invention (such as cancer, such as the EGFR-related cancer). Non-limiting examples include human, cow, rat, mouse, cat, dog, monkey, goat, sheep, and other non-mammals. In a preferred embodimen, the subject is the human.

In another aspect, the present invention provides a pharmaceutical composition, which comprises a therapeutically effective amount of the therapeutic T cell of the present invention, and a pharmaceutically acceptable carrier. In some embodiments, the pharmaceutical composition is used to treat EGFR-related cancer in the subject.

The "pharmaceutically acceptable carrier" used herein include any and all physiologically compatible solvents, dispersion media, coatings, antibacterial and antifungal agents, isotonic agents, and absorption retardants and the like. Preferably, the carrier is suitable for intravenous, intramuscular, subcutaneous, parenteral, spinal or epidermal administration (such as by injection or infusion).

In another aspect, the present invention provides a use of the therapeutic T cell of the present invention in preparation of a drug for treating EGFR-related cancer.

In another aspect of the present invention, a method for treating EGFR-related cancer is further provided, including administering a therapeutically effective amount of the therapeutic T cell of the present invention or the pharmaceutical composition of the present invention to a subject in need.

In some implementation modes, the method further includes administering radiation therapy and/or chemotherapy and/or another tumor targeted drug (such as a monoclonal antibody or a small molecule compound targeting other antigens) and/or immunotherapy (such as an immune checkpoint inhibitor) to the subject.

As used herein, the "therapeutically effective amount" or "therapeutically effective dose" or "effective amount" refers to an amount of substance, compound, material or cells at least sufficient to produce the therapeutic effect after being administered to the subject. Therefore, it is the amount necessary to prevent, cure, improve, block or partially block a disease or symptoms of the disease. As used herein, the treatment also encompasses preventing the recurrence of the disease (such as cancer).

For example, the "effective amount" of the cell or pharmaceutical composition of the present invention preferably leads to a decrease in the severity of the disease symptoms, an increase in the frequency and duration of an asymptomatic period of the disease, or the prevention of damage or disability caused by disease pain. For example, for the treatment of tumors, relative to an untreated subject, the "effective amount" of the cell or pharmaceutical composition of the present invention preferably inhibits the tumor cell growth or tumor growth by at least about 10%, preferably at least about 20%, more preferably at least about 30%, more preferably at least about 40%, more preferably at least about 50%, more preferably at least about 60%, more preferably at least about 70%, more preferably at least about 80%, and more preferably at least about 90%. The ability to inhibit the tumor growth may be evaluated in an animal model system that predicts the therapeutic effect on human tumors. Alternatively, it may be evaluated by examining the ability to inhibit the tumor cell growth, and this inhibition may be determined in vitro by experiments well-known to those skilled in the art.

In practical applications, the dose level of cells in the pharmaceutical composition of the present invention may be changed, to obtain the amount of active ingredients composition, and administration mode that may effectively achieve the desired therapeutic response to the specific subject, without the toxicity to a patient. The dose level can be selected depends on a variety of pharmacokinetics factors, including the activity of the specific composition of the present invention used, the route of administration, the timing of administration, the excretion rate of the specific compound used, the duration of treatment, other drugs, compounds and/or materials used in combination with the specific composition used, the age, gender, weight, status, overall health conditions and medical history of the subject receiving the treatment, and similar factors well-known in the medical field.

As used herein, the therapeutically effective amount of the therapeutic T cells refers to an amount of the therapeutic T cells that may reduce the load of tumor cells after use, such as an amount that may reduce the load of the tumor cells by at least about 5%, at least about 10%, at least about 20%, at least about 30%, at least about 40%, at least about 50%, at least about 60%, at least about 70%, at least about 80%, and at least about 90%, or achieve the complete remission of the cancer. In some embodiments of each aspect of the present invention, the effective amount of the therapeutic T cells is about 10⁴ to about 10⁹ cells, such as about 10⁴, about 10⁵, about 10⁶, about 10⁷, about 10⁸, or about 10⁹ cells. In some embodiments, the administration amount of the therapeutic T cells is determined according to the subject's weight, such as about 10⁴ cells/kg weight to about 10⁹ cells/kg weight, such as about 10⁴, about 10⁵, about 10⁶, about 10⁷, about 10⁸, or about 10⁹ cells/kg weight.

It is indicated from research results of the present inventor that the therapeutic T cell with the TGFβ receptor (such as TGFBRI, TGFBRII, and TGFBRIII) knocked down or knocked out of the present invention, relative to the control T cell (the TGFβ receptor (such as TGFBRI, TGFBRII, and TGFBRIII) is not knocked down or knocked out), may achieve better therapeutic effect at the lower dose. For example, the therapeutic T cell with the TGFβ receptor (such as TGFBRI, TGFBRII, and TGFBRIII) of the present invention may achieve the better tumor killing effect than the control T cell at lower effect-target ratio and/or for longer time. This is particularly beneficial for reducing the preparation time and cost, while the side effects caused by high-dose administration may be reduced.

For example, the administration dose of the therapeutic T cell with the TGFβ receptor (such as TGFBRIII) of the present invention is about 2 times, about 3 times, about 4 times, about 5 times, about 6 times, about 7 times, about 8 times, about 9 times, about 10 times, about 15 times, about 20 times, about 30 times, about 40 times, about 50 times, about 80 times, about 100 times, about 150 times, about 160 times, about 200 times or more times lower than the administration dose of the control T cell that the TGFβ receptor (such as TGFBRII) is not knocked down or knocked out.

The administration of the cell or composition according to the present invention may be performed in any convenient manners, including injection, infusion, implantation, or transplantation. The administration of the cell or composition described herein may be administered intravenously, lymphatically, intradermally, intratumorally, intramedullarily, intramuscularly, or intraperitoneally. In one implementation scheme, the cell or composition of the present invention is preferably administered by intravenous injection.

In some embodiments of each aspect of the present invention, the EGFR-related cancer is a cancer in which tumor cells express EGFR, including but not limited to esophageal cancer, gastric cancer, colon cancer, rectal cancer, colorectal cancer, pancreatic cancer, lung cancer (including non-small cell lung cancer NSCLC), breast cancer, cervical cancer, corpus cancer, endometrial cancer, ovarian cancer, bladder cancer, head and neck cancer (including head and neck squamous cell cancer SCCHN), osteosarcoma, prostate cancer, neuroblastoma, renal cancer, glioma, glioblastoma and skin cancer (including epithelial cancer).

In another aspect, the present invention provides a method for preparing the therapeutic T cell of the present invention, and the method includes the following steps:
a) providing an isolated T cell; and
b) introducing the polynucleotide of the present invention or the expression construct of the present invention into the T cell, thereby causing the T cell to express the CAR of the present invention.

The step of providing the isolated T cell may be performed by methods known in the art for separating the T cell. For example, a commercial kit may be used to isolate the T cell from the peripheral blood of the subject. The suitable kit includes but not limited to an EasySep human T cell enrichment kit (Stemcell Technologies). As described above, the isolated T cells may not necessarily be homogeneous, but may be a mixed population of different cells, and preferably the T cells are enriched in the population.

In some embodiments, the method further includes a step:
x) knocking down or knocking out the expression of the TGFP receptor (such as TGFBRI, TGFBRII, and TGFBRIII) in the T cell.

In some embodiments, the step x) is performed before the step b). In some embodiments, the step x) is performed after the step b).

A plurality of methods for knocking down or knocking out protein expression in cells is known in the art. In some embodiments, the expression of the TGFβ receptor (such as TGFBRII) in the T cell is knocked down or knocked out by introducing antisense RNA, antagomir, siRNA, and shRNA. In other implementation modes, the expression of the TGFβ receptor (such as TGFBRII) in the T cell is knocked down or knocked out by a method of gene editing, for example, by introducing a Meganuclease, zinc finger nuclease, transcription activator like effector nuclease or CRISPR system. In preferred embodiments of the method of the present invention, the CRISPR system is used to knock down or knock out the expression of the TGFβ receptor (such as TGFBRII) in the T cell. In some implementation modes, the nuclease (CRISPR nuclease) used by the CRISPR system may be selected from Cas3, Cas8a, Cas5, Cas8b, Cas8c, Cas10d, Cse1, Cse2, Csy1, Csy2, Csy3, GSU0054, Cas10, Csm2, Cmr5, Cas10, Csx11, Csx10, Csf1, Cas9, Csn2, Cas4, Cpf1, C2c1, C2c3 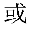 C2c2 proteins, or functional variants of these nucleases, for example.

The polynucleotide, expression construct and/or protein may be introduced into the cells by any appropriate methods, including electroporation; transfection using calcium chloride, rubidium chloride, calcium phosphate, DEAE-glucan or other substances; particle bombardment; liposome transfection; and infection (for example, the expression construct is a virus).

The T cell of the present invention may be activated and amplified before or after any modification steps. The T cell may be amplified in vitro or in vivo.

Therefore, in some embodiments, the method further includes a step:
y) amplifying the T cell.

In some embodiments, the step y) is performed before and/or after the step b). In some embodiments, the step y) is performed before and/or after the step x).

Typically, the T cell of the present invention may be amplified, for example, by contacting a reagent that stimulates a co-stimulatory molecule on the surface of CD3 TCR complex and T cell to generate a T cell activation signal. For example, chemicals such as calcium ionophore A23187, phorbol 12-myristate 13-acetate (PMA), or mitotic lectin such as phytohemagglutinin (PHA) may be used to generate activation signals of the T cell. In some embodiments, the T cell may be activated in vitro by contacting, for example, an anti-CD3 antibody or an antigen binding fragment thereof, or an anti-CD2 antibody immobilized on the surface, or by contacting a protein kinase C activator (for example, bryostatin) with the calcium ionophore. For example, under conditions suitable for stimulating T cell proliferation, the T cell may contact the anti-CD3 antibody and the anti-CD28 antibody. The conditions applicable to T cell culture include suitable media that may contain factors necessary for proliferation and vitality (such as Minimal Essential Media or RPMI Media 1640, or X-vivo 5, (Lonza)), herein the necessary factors include serum (such as fetal bovine or human serum), interleukin-2 (IL-2), insulin, IFN-y, IL-4, IL-7, GM-CSF, IL-10, IL-2, IL-15, TGFβ and TNF, or additives known to those skilled in the art for cell growth. Other additives used for the cell growth include but not limited to surfactants, human plasma protein powder, and reducing agents such as N-acetylcysteine and 2-mercaptoacetic acid. The medium may include RPMI 1640, A1M-V, DMEM, MEM, a-MEM, F-12, X-Vivo 1 and X-Vivo 20, Optimizer, amino acids, sodium pyruvate and vitamins, serum-free or moderately supplemented serum (or plasma) or a set of definite hormones, and/or a certain amount of cytokines sufficient to promote T cell growth and amplification. The T cell may be maintained under the necessary conditions to support the growth, such as appropriate temperature (for example, 37°C) and environment (for example, air plus 5% CO₂).

In another aspect, the present invention further provides a kit for preparing the therapeutic T cell of the present invention. The kit of the present invention includes the polynucleotide of the present invention, the expression construct of the present invention, and/or the tools for knocking down or knocking out the expression of the TGFβ receptor (such as TGFBRII) such as antisense RNA, antagomir, siRNA, shRNA, a Meganuclease, zinc finger nuclease, transcription activator like effector nuclease or CRISPR system or its encoding nucleic acid or vector. The kit may further include a reagent for separating, culturing, and/or amplifying the T cell, a preparation for introducing the polynucleotide or protein into the cell, and the like.

### Examples

The present invention is further described below by means of examples, but it is not limited to the scope of the examples described.

### Experimental materials and methods

### 1. Separation, stimulation, and amplification of CD3+ T cell

Fresh umbilical cord blood from healthy donor sources was acquired from the Beijing Umbilical Cord Blood Bank, with consent informed. Mononuclear cells were separated by using human lymphocyte separation solution (Tianjin Haoyang Biological Products Technology Co., Ltd.). The T cells were separated by using an EasySep human T cell enrichment kit (Stemcell Technologies), and anti-CD3/CD28 Dynabeads (Thermo Fisher Scientific) were added according to use instructions, to activate the separated T cells in a ratio of 1:1. The medium for the T cell was X-VIVO15 medium (Lonza), with 5% (v/v) heat inactivated fetal bovine serum (GIBCO) and 400 IU/mL recombinant human IL-2 (Sino-biological Inc.) added.

### 2. Preparation of anti-EGFR CAR-T cell

Seven anti-EGFR ScFv gene fragments were synthesized respectively (Huada Qinglan Biotechnology Co., Ltd.), and the ScFv fragments were respectively cloned into a pRRLSIN lentivirus vector by two enzyme digestion sites of BamH I and Mlu I. A three-plasmid system vector plasmid, pMD2.G and psPAX2 were co-transfected into 293T cells by using Lipo3000 (Thermo Fisher Scientific), and lentivirus culture supernatant was collected at 48 hours and 72 hours respectively, and concentrated with a concentration column (Millipore, Amicon Ultra-15 centrifuge Filters, Ultracel-100K). After human primary CD3+ T cells were stimulated by magnetic beads for 24 hours, lentivirus infection was performed. When infected, the CD3+ T cell density was adjusted to 2×106/ml. According to a ratio of MOI=1, a co-transfection reagent Polybrene (Sigma) was added at the same time, the final concentration of Polybrene was 10 µg/ml. After 48 h of the infection, the positive rate of CAR-T cells was detected by a flow cytometer.

### 3. Flow cytometry detection

About 1-10×10⁵ cells were collected, and stained according to the recommended dosage of the antibody company. CytoFLEX (Beckman Coulter Inc.) was used for on-machine detection. The following antibodies were used: goat anti human IgG (H+L) flow cytometry antibody Alexa Fluor 647 (109-606-003, Jackson), mouse anti human CD3 flow cytometry monoclonal antibody Brilliant Violet 421 (300434, BioLegend), mouse anti human EGFR monoclonal antibody PE (352903, BioLegend), 806 recombinant monoclonal antibody (synthesized by Genscript), mouse anti human CD4 flow cytometry monoclonal antibody PE (300508, BioLegend), mouse anti human CD8α flow cytometry monoclonal antibody APC (301014, BioLegend), mouse anti human CD45RO flow cytometry monoclonal antibody PE (304205, BioLegend), and mouse anti human CCR7 flow cytometry monoclonal antibody APC (353213, BioLegend).

### 4. Electroporation of CAR-T cell and editing efficiency detection

Three days after T cell activation, Dynabeads were removed with magnet. Before electroporation, RNP complex was prepared, 6 µg of Cas9 protein (Shenzhen Feipeng Biotechnology Co., Ltd.) and 6 µg of sgRNA (target sequence: CCTGAGCAGCCCCCGACCCA) transcribed and prepared in vitro were incubated at room temperature for 20 minutes. 1×10⁶ CAR-T cells were resuspended by using 20 µl of P3 Primary Cell 4D-NucleofectorX Kit electroporation solution (V4XP-3024, Lonza), and incubated RNP was added. A 4D-Nucleofector System N (Lonza) electroporator was used for the electroporation under EO-115 electroporation conditions. After the electroporation, cell mixture was extracted, and transferred to a preheated T cell medium. After 48 hours, the electroporation efficiency was detected. Sanger sequencing (sequencing primer: TGFbR2-TIDE-F: 5'-cacatctggcccgcacatct-3'; and TGFbR2-TIDE-R: 5'-ggaaactttcctcgtttccgc-3') was performed on the PCR product (primer: TGFbR2-TIDE-F: 5'-cacatctggcccgcacatct-3'; and TGFbR2-GT-R: 5'-gggtggctcagaaagagctg-3') of Surveyor assay. By a website http://tide.nki.nl, sequencing results were analyzed.

### 5. CAR-T cell in vitro killing experiment (Luciferase detection method)

Construction of CRL-5826-Luci cell: wild-type CRL-5826 cells were infected with a lentivirus expressing luciferase and Puromycin resistance screening gene, and then screened with Puromycin for two weeks, to obtain CRL-5826-Luci cells stably expressing the luciferase. Killing experiment: target cells CRL-5826-Luci were resuspended by using a 1640 complete medium, so that the cell density was 1×10⁶/ml. Target cell suspension was inoculated in a 96-well plate, 100 ul per well. Different numbers of effector CAR-T cells were respectively added according to different effect-target ratios, and 4 replicates were made for each effect-target ratio. The final volume of each well was 200µl. They were placed in an incubator, and taken out at different time points to detect the killing efficiency. During detection, 10 µl of a Steady-glo fluorescein substrate (Promega) was added to each well, and reacted for 5 minutes. The fluorescence value was detected by using a microplate reader PerinElmer VICTOR X3. The killing efficiency of the effector cells to the target cells was calculated based on the fluorescence value of each well: specific lysis (%)=(1-RUL effector cell plus target cell/RUL target cell)×100 (RUL: relative light unit).

### 6. CAR-T cell in vitro killing experiment (RTCA detection method)

The in vitro killing function of effector CAR-T cells to target cells was detected by using a real-time unlabeled cell function analyzer. Fibroblast cells, CRL-5826 cells, and K562 cells were respectively seeded into E-Plate16 (ACEA), 2500 cells were added to each well, and two replicates were made. After 24 hours, the effector CAR-T cells were respectively added according to different effect-target ratios (0.2:1, 0.05:1, 0.0125:1, and 0:1). The Cell Index (CI) value was continuously monitored for 4 days. The killing efficiency of effector cells to target cells was calculated based on the CI value of each well: specific lysis (%)=(1-CI effector cell plus target cell/CI target cell)×100.

### 7. Multi-round antigen stimulation (Stress-Test) experiment

2×10⁵ CAR-T cells were co-cultured with CRL-5826 tumor cells, and the effect-target ratio is 1:1. After two days, all tumor cells were lysed, and after the CAR-T cells were counted, and new tumor cells were added. By analogy, the new tumor cells were added every other day, and the effect-target ratio was kept at 1:1, until there was a significant difference in the killing efficiency of the CAR-T cells among different groups. The TGF-β1 concentration in an addition group was maintained at 5 ng/ml.

### 8. Detection of CAR-T cell function in tumor-bearing mouse model

Experimental mice were six-week-old NPG female mice (purchased from Weitongda Company). The CRL-5826 cells were resuspended with DPBS, and the cell density was 2×10⁷/ml. 100 ul of cell suspension was respectively taken, and 100 µl of Matrigel was added, subcutaneous injection was performed on the mice. Each mouse was injected with about 2×10⁶ CRL-5826 cells, and the tumor volume is about 300 mm3 after 4 weeks. According to the size of tumors, the tumor-bearing mice were randomly grouped, and 5 mice were assigned per experimental group. The CAR-T cells were injected into the tail vein once at different injection doses (CAR+ is about 50%). The tumor volume, the human CD3 content in peripheral blood, and the proportion of T cell subtypes were measured every week. Tumor block re-inoculation: mice in a PBS group were euthanized, and tumor blocks were taken out, divided into 200-300 mm3 tumor blocks, and respectively inoculated subcutaneously on the opposite side of the mice in which the tumors were completely cleared. Four new NPG mice were taken, and inoculated with the divided tumor blocks subcutaneously as a re-inoculation control.

### Example 1: Synthesis of anti-EGFR ScFv sequence and vector construction

Six humanized anti-EGFR ScFv sequences and one mouse-derived anti-EGFR ScFv were found from existing patents and National Center of Biotechnology Information (NCBI), namely hu806 (US009493568B2), E2 (US20150030599A1), Pan (US20150152184A1), Nec (WO2005/090407A1), Nimo (US6506883B2), 301 (GeneBank JQ306330.1), and m806 (WO02092771A2). After gene synthesis, these seven ScFv were respectively inserted into a lentivirus vector pRRLSIN plasmid with a CAR framework gene (Fig. 1).

### Example 2: Preparation of anti-EGFR CAR-T cell

The seven CAR structures containing different ScFv described in Example 1 were respectively introduced into human primary T cells by lentiviruses. After the human primary T cells were infected at the same viral titer, the positive rate of the CAR-T cells was detected on Day 5 post infection (Fig. 2). From results, it may be seen that even under the condition of the same lentivirus titer, the positive rates of the different CAR-T cells still have relatively large differences and clusters.

### Example 3: Comparison of in vitro and in vivo killing functions of anti-EGFR CAR-T cell

In order to compare the killing functions of anti-EGFR CAR-T cells from different scFv sources, the CRL-5826 cells were subjected to in vitro low effect-target ratio long-term killing experiment and tumor antigen continuous stimulation killing Stress-Test experiment (Fig. 3, and Fig. 4). From results, it may be seen that hu806 and Nimo CAR-T have the stronger in vitro tumor killing function compared to the other 4 types of scFv CAR-T. In addition, it is indicated from Stress-Test results that the tumor clearance effect of hu806 CAR-T is superior to that of Nimo CAR-T cells. Next, NPG mouse in vivo tumor-bearing and treatment experiment (Fig. 5A) was performed. CRL-5826 cells subcutaneously formed tumors, and after 5 weeks, 6 types of anti-EGFR CAR-T cells were respectively injected into the tail vein at the same dose. After that, changes in tumor volume were observed every week (Fig. 5B). It is indicated from results that the hu806 CAR-T cells have the optimal in vivo tumor clearance effect.

### Example 4: Comparison of in vitro killing functions of humanized hu806 CAR-T and mouse-derived m806 CAR-T cells

The anti-EGFR monoclonal antibody 806 ScFv was originally derived from mouse IgG2b (m806), and the FR region sequence was humanized to become humanized 806 (hu806). In this applicaiton, the in vitro functions of CAR-T cells derived from humanized and mouse 806 ScFv were compared. It is indicated from experimental results that the hu806 CAR-T cells have the stronger in vitro anti-tumor function (Fig. 6).

The amino acid sequence and the nucleotide sequence of mouse IgG2b (m806) CAR are respectively shown in SEQ ID NOs: 81 and 82, and respective portions of the mouse IgG2b (m806) CAR correspond to Table 1 below.

**Table 1: Regions in mouse IgG2b (m806) CAR**

| Region | Corresponding to nucleotide in SEQ ID NO: 82 |
|---|---|
| BamH1 | 1-6bp |
| CD8α signal peptide | 7-69bp |
| m806 ScFv | 70-786bp |
| Mlu I | 787-792bp |
| CD8α hinge region | 793-924bp |
| EcoR V | 925-930bp |
| CD8α transmembrane region | 931-999bp |
| 4-1BB co-stimulatory domain | 1000-1125bp |
| CD3ζ | 1126-1464bp |
| Sal I | 1465-1470bp |

### Example 5: Anti-tumor ability can be enhanced after TGF-β Receptor II is knocked out in hu806 CAR-T cells

The anti-tumor functions of hu806 CAR-T cells in which TGF-β Receptor was knocked out and was not knocked out were compared. Human primary T cells were infected with lentiviruses, and after 48 hours, Cas9 RNPs targeting TGFbR2 were electroporated. After two days, genomic DNA of knocked-out cells was extracted, and the knockout efficiency (Fig. 7A) and the positive rate (Fig. 7B) of CAR-T cells were detected by a TIDE method. After 7 days of in vitro culture, the in vitro tumor killing conditions of hu806 CAR-T cells and hu806-TKO CAR-T cells in the presence of TGF-β are observed.

It is indicated from results that TGF-β inhibits the in vitro anti-tumor function of the hu806 CAR-T cells, and after the TGF-β Receptor II was knocked out, the inhibiting effect (Fig. 8A) of TGF-β on the CAR-T cell function can be reversed. It is also indicated from the Stress-Test experimental results that after a plurality of rounds of tumor antigen continuous stimulation, hu806-TKO had the more anti-tumor advantages compared to the hu806 CAR-T cells (Fig. 8B). In addition, the hu806-TKO CAR-T cells in which the TGF-β Receptor II was knocked out have the more proliferation advantages than the hu806 CAR-T cells (Fig. 8C).

### Example 6: NPG mouse in vivo experiment shows that hu806-TKO CAR-T has the better therapeutic effect

Hu806 CAR-T and 806-TKO CAR-T cells were injected at different doses, to observe changes in tumor volume in tumor-bearing NPG mice (Fig. 9A). It is indicated from animal in vivo experimental results that the injection dose is higher, and the tumor clearance speed is faster. In addition, under the same dose conditions, the therapeutic effect of the hu806-TKO CAR-T cells was significantly better than that of the hu806 CAR-T cells (Fig. 9A). Tumor re-inoculation was performed on the mice in the hu806-TKO group in which tumors were completely cleared. After 3-4 weeks, the experimental group mice showed the ability to clear the tumors again. By analyzing the proportion of human CD3 in peripheral blood of the mice, it can be seen that the proportion of hCD3 in the hu806-TKO group was significantly higher than that in the hu806 group (Fig. 8B), and it is positively correlated with the tumor clearance effect.

### Example 7: Proportion of T cell subtypes in peripheral blood of tumor-bearing NPG mice using hu806-TKO CAR-T cells

In order to observe the amplification and subtype proportion changes of CAR-T cells infused into animals, hu806 and hu806-TKO cells were prepared by using # 4 donor CD3 T cells with good in vivo amplification effect. Two types of CAR-T cells and PBS control were injected into the tail veins, and the injection dose was 5e6 CAR+/mouse. Blood was collected every week to observe T cell subtypes. It is indicated again from results that the knockout group had better tumor clearance effect (Fig. 10A). The proportion of hCD3 in the peripheral blood of mice was first increased and then decreased. In the later stage of treatment, the hu806-TKO group still maintained a higher proportion of hCD3 compared to the hu806 group (Fig. 10B). Further analysis of the human CD3 subtypes in the mouse peripheral blood shows that the TKO group had a higher proportion of memory state T cells, especially the central memory T cell proportion, which is more advantageous (Fig. 10C). According to documents published, the proportion of the central memory T cells is positively correlated with prognosis and efficacy. From CD4 and CD8 staining results, it can be seen that in the early stage of treatment, the proportion of CD8 T cells was higher in the TKO group. Over time, CD4 T cells became a main cell subgroup (Fig. 10D).

### Example 8: Exploration of therapeutic dose for hu806-TKO CAR-T cells

In order to provide injection therapeutic dose reference for clinical experiments, a dose grouping experiment was performed on tumor-bearing NPG mice. Different infusion doses were formulated based on the equivalent dose conversion between mice and humans (Table 2). From results of in vivo grouping therapy in animals, it can be seen that all four dose groups of hu806-TKO could effectively clear CDX model tumors, and the dose was related to the tumor clearance speed (Fig. 11A). After tumor clearance, two therapeutic dose groups of tumor re-inoculation experiments were conducted, and effective reduction in the volume of re-inoculated tumors was observed in both groups (Fig. 11A). The hCD3 content in the mouse peripheral blood was related to the infusion dose, and along with the treatment time, the hCD3 content was first increased and then decreased (Fig. 11B).

**Table 2: Dose table of Hu806-TKO CAR-T cells for in vivo treatment of tumor-bearing NPG mice**

| **NPG mouse dose experimental grouping** | | | | |
|---|---|---|---|---|
| **Group** | **Treatment** | **Dose Level^{a}** | **Human equivalent dose**^{a} | **Times of administration^{b}** |
| 1 | T cell | 2 ×10⁶ cells/mouse | - | single dose |
| 2 | hu806-TKO CAR⁺T | 2 ×10⁶ cells/mouse (1×10⁸ cells/kg) | 1×10⁷ cells/kg | single dose |
| 3 | hu806-TKO CAR⁺T | 1 ×10⁶ cells/mouse (5×10⁷ cells/kg) | 5×10⁶ cells/kg | 1-3 doses |
| 4 | hu806-TKO CAR⁺T | 0.5 ×10⁶ cells/mouse (2.5×10⁷ cells/kg) | 2.5×10⁶ cells/kg | 1-3 doses |
| 5 | hu806-TKO CAR⁺T | 0.25 ×10⁶ cells/mouse (1.25×10⁷ cells/kg) | 1.25×10⁶ cells/kg | 1-3 doses |
| 6 | PBS | | | single dose |

| | | | | |
|---|---|---|---|---|
| a. The weight of a mouse is calculated as 20 g, and the human equivalent dose is converted by a coefficient of 10 (mouse: human). b. The lower dose groups are administered for many times depending on the tumor inhibition conditions, and the interval of administration is determined based on results of peripheral blood flow cytometry. | | | | |

### Example 9: In vivo safety of hu806 CAR-T cells

The main risk of CAR-T cell therapy lies in the off-target effect. In order to detect the off-target effect of anti-EGFR hu806 ScFv and clarify its safety in vivo, a hu806 recombinant antibody was used to stain lung squamous cell carcinoma cells and human primary fibroblasts. From flow cytometry results, it can be seen that both CRL-5826 and fibroblasts expressed EGFR, while the fibroblasts only expressed a small amount of hu806 antigens. Blood-derived leukemia cells K562 did not express EGFR (Fig. 12A). Correspondingly, in vitro killing detection on these three types of cells showed that hu806 CAR-T cells had the strong killing function against EGFR positive 806 antigen positive CRL-5826 cells, while they almost did not kill EGFR positive 806 antigen negative fibroblasts and EGFR negative K562 cells (Fig. 12B). This suggests that the risk of off-target side effects is lower when the hu806 cells are injected into a body as a drug.

### Sequences:

| | |
|---|---|
| SEQ ID NO:1 | hu806 VH-CDR1 |
| SDFAWN | |
| SEQ ID NO:2 | hu806 VH-CDR2 |
| YISYSGNTRYQPSLKS | |
| SEQ ID NO:3 | hu806 VH-CDR3 |
| VTAGRGFPY | |
| SEQ ID NO:4 | hu806 VL-CDR1 |
| HSSQDINSNIG | |
| SEQ ID NO:5 | hu806 VL-CDR2 |
| HGTNLDD | |
| SEQ ID NO:6 | hu806 VL-CDR3 |
| VQYAQFPWT | |
| SEQ ID NO:7 | hu806 VH |
| QVQLQESGPGLVKPSQTLSLTCTVSGYSISSDFAWNWIRQPPGKGLEWMGYISYSGNTRY QPSLKSRITISRDTSKNQFFLKLNSVTAADTATYYCVTAGRGFPYWGQGTLVTVSS | |
| SEQ ID NO:8 | hu806 VL |
| DIQMTQSPSSMSVSVGDRVTITCHSSQDINSNIGWLQQKPGKSFKGLIYHGTNLDDGVPSR FSGSGSGTDYTLTISSLQPEDFATYYCVQYAQFPWTFGGGTKLEIKR | |
| SEQ ID NO:9 | hu806 ScFv |
| QVQLQESGPGLVKPSQTLSLTCTVSGYSISSDFAWNWIRQPPGKGLEWMGYISYSGNTRY QPSLKSRITISRDTSKNQFFLKLNSVTAADTATYYCVTAGRGFPYWGQGTLVTVSSGGGG SGGGGSGGGGSDIQMTQSPSSMSVSVGDRVTITCHSSQDINSNIGWLQQKPGKSFKGLIYH GTNLDDGVPSRFSGSGSGTDYTLTISSLQPEDFATYYCVQYAQFPWTFGGGTKLEIKR | |
| SEQ ID NO:10 | E2 VH-CDR1 |
| NYDM | |
| SEQ ID NO:11 | E2 VH-CDR2 |
| GISHSSGSKYYADSVKG | |
| SEQ ID NO:12 | E2 VH-CDR3 |
| KDATPRPLKPFDY | |
| SEQ ID NO:13 | E2 VL-CDR1 |
| TGSSSNIGNNDVS | |
| SEQ ID NO:14 | E2 VL-CDR2 |
| DDNKRPS | |
| SEQ ID NO:15 | E2 VL-CDR3 |
| GSWDASLNA | |
| SEQ ID NO:16 | E2 VH |
| EVQLLESGGGLVQPGGSLRLSCAASGFTFSNYDMSWVRQAPGKGLEWVSGISHSSGSKY YADSVKGRFTISRDNSKNTLYLQMNSLRAEDTAVYYCAKDATPRPLKPFDYWGQGTLVT VSS | |
| SEQ ID NO:17 | E2 VL |
| QSVLTQPPSASGTPGQRVTISCTGSSSNIGNNDVSWYQQLPGTAPKLLIYDDNKRPSGVPD RFSGSKSGTSASLAISGLRSEDEADYYCGSWDASLNAYVFGGGTKLTVLG | |
| SEQ ID NO:18 | E2 ScFv |
| EVQLLESGGGLVQPGGSLRLSCAASGFTFSNYDMSWVRQAPGKGLEWVSGISHSSGSKY YADSVKGRFTISRDNSKNTLYLQMNSLRAEDTAVYYCAKDATPRPLKPFDYWGQGTLVT VSSGGGGSGGGGSGGGGSQSVLTQPPSASGTPGQRVTISCTGSSSNIGNNDVSWYQQLPG TAPKLLIYDDNKRPSGVPDRFSGSKSGTSASLAISGLRSEDEADYYCGSWDASLNAYVFG GGTKLTVLG | |
| SEQ ID NO:19 | Pan VH-CDR1 |
| SGDYYWT | |
| SEQ ID NO:20 | Pan VH-CDR2 |
| HIYYSGNTNYNPSLKS | |
| SEQ ID NO:21 | Pan VH-CDR3 |
| DRVTGAFDI | |
| SEQ ID NO:22 | Pan VL-CDR1 |
| QASQDISNYLN | |
| SEQ ID NO:23 | Pan VL-CDR2 |
| DASNLET | |
| SEQ ID NO:24 | Pan VL-CDR3 |
| QHFDHLPLA | |
| SEQ ID NO:25 | Pan VH |
| QVQLQESGPGLVKPSETLSLTCTVSGGSVSSGDYYWTWIRQSPGKGLEWIGHIYYSGNTN YNPSLKSRLTISIDTSKTQFSLKLSSVTAADTAIYYCVRDRVTGAFDIWGQGTMVTVSSAS TKGPSVFPLAPCSR | |
| SEQ ID NO:26 | Pan VL |
| | |
| SEQ ID NO:27 | Pan ScFv |
| QVQLQESGPGLVKPSETLSLTCTVSGGSVSSGDYYWTWIRQSPGKGLEWIGHIYYSGNTN YNPSLKSRLTISIDTSKTQFSLKLSSVTAADTAIYYCVRDRVTGAFDIWGQGTMVTVSSAS TKGPSVFPLAPCSRGGGGSGGGGSGGGGSDIQMTQSPSSLSASVGDRVTITCQASQDISNY LNWYQQKPGKAPKLLIYDASNLETGVPSRFSGSGSGTDFTFTISSLQPEDIATYFCQHFDH LPLAFGGGTKVEIK | |
| SEQ ID NO:28 | Nec VH-CDR1 |
| SGDYYWS | |
| SEQ ID NO:29 | Nec VH-CDR2 |
| YIYYSGSTDYNPSLKS | |
| SEQ ID NO:30 | Nec VH-CDR3 |
| VSIFGVGTFDY | |
| SEQ ID NO:31 | Nec VL-CDR1 |
| RASQSVSSYLA | |
| SEQ ID NO:32 | Nec VL-CDR2 |
| DASNRAT | |
| SEQ ID NO:33 | Nec VL-CDR3 |
| HQYGSTPLT | |
| SEQ ID NO:34 | Nec VH |
| | |
| SEQ ID NO:35 | NecVL |
| | |
| SEQ ID NO:36 | Nec ScFv |
| QVQLQESGPGLVKPSQTLSLTCTVSGGSISSGDYYWSWIRQPPGKGLEWIGYIYYSGSTDY NPSLKSRVTMSVDTSKNQFSLKVNSVTAADTAVYYCARVSIFGVGTFDYWGQGTLVTVS SGGGGSGGGGSGGGGSEIVMTQSPATLSLSPGERATLSCRASQSVSSYLAWYQQKPGQAP RLLIYDASNRATGIPARFSGSGSGTDFTLTISSLEPEDFAVYYCHQYGSTPLTFGGGTKAEI K | |
| SEQ ID NO:37 | Nimo VH-CDR1 |
| RSSQNIVHSNGNTYLD | |
| SEQ ID NO:38 | Nimo VH-CDR2 |
| KVSNRFS | |
| SEQ ID NO:39 | Nimo VH-CDR3 |
| FQYSHVPWT | |
| SEQ ID NO:40 | Nimo VL-CDR1 |
| NYYIY | |
| SEQ ID NO:41 | Nimo VL-CDR2 |
| GGINPTSGGSNFNEKFKT | |
| SEQ ID NO:42 | Nimo VL-CDR3 |
| QGLWFDSDGRGFDF | |
| SEQ ID NO:43 | Nimo VH |
| QVQLQQSGAEVKKPGSSVKVSCKASGYTFTNYYIYWVRQAPGQGLEWIGGINPTSGGSN FNEKFKTRVTITVDESTNTAYMELSSLRSEDTAFYFCARQGLWFDSDGRGFDFWGQGST VTVSS | |
| SEQ ID NO:44 | Nimo VL |
| DIQMTQSPSSLSASVGDRVTITCRSSQNIVHSNGNTYLDWYQQTPGKAPKLLIYKVSNRFS GVPSRFSGSGSGTDFTFTISSLQPEDIATYYCFQYSHVPWTFGQGTKLQITRE | |
| SEQ ID NO:45 | Nimo ScFv |
| DIQMTQSPSSLSASVGDRVTITCRSSQNIVHSNGNTYLDWYQQTPGKAPKLLIYKVSNRFS | |
| GVPSRFSGSGSGTDFTFTISSLQPEDIATYYCFQYSHVPWTFGQGTKLQITREGGGGSGGG GSGGGGSQVQLQQSGAEVKKPGSSVKVSCKASGYTFTNYYIYWVRQAPGQGLEWIGGIN PTSGGSNFNEKFKTRVTITVDESTNTAYMELSSLRSEDTAFYFCARQGLWFDSDGRGFDF WGQGSTVTVSS | |
| SEQ ID NO:46 | 301 VH CDR1 |
| GTFSSYA | |
| SEQ ID NO:47 | 301 VH CDR2 |
| IIPIFGTA | |
| SEQ ID NO:48 | 301 VH CDR3 |
| ARTRLKHQ | |
| SEQ ID NO:49 | 301 VL CDR1 |
| SLRSYY | |
| SEQ ID NO:50 | 301 VL CDR2 |
| GKNN | |
| SEQ ID NO:51 | 301 VL CDR3 |
| NSRDSSGPV | |
| SEQ ID NO:52 | 301 VH |
| QVQLVQSGAEVKKPGSSVKVSCKASGGTFSSYAISWVRQAPGQGLEWMGGIIPIFGTANY AQKFQGRVTITADESTSTAYMELSSLRSEDTAVYYCARTRLKHQWGQGTLVTVSS | |
| SEQ ID NO:53 | 301 VL |
| LSSELTQDPAVSVALGQTVRITCQGDSLRSYYASWYQQKPGQAPVLVIYGKNNRPSGIPD RFSGSSSGNTASLTITGAQAEDEADYYCNSRDSSGPVFGGGTKLTVLGAAA | |
| SEQ ID NO:54 | 301 ScFv |
| MAQVQLVQSGAEVKKPGSSVKVSCKASGGTFSSYAISWVRQAPGQGLEWMGGIIPIFGT ANYAQKFQGRVTITADESTSTAYMELSSLRSEDTAVYYCARTRLKHQWGQGTLVTVSSG GGGSGGGGSGGSALSSELTQDPAVSVALGQTVRITCQGDSLRSYYASWYQQKPGQAPVL VIYGKNNRPSGIPDRFSGSSSGNTASLTITGAQAEDEADYYCNSRDSSGPVFGGGTKLTVL GAAA | |
| SEQ ID NO:55 | CD8α signal peptide |
| MALPVTALLLPLALLLHAARP | |
| SEQ ID NO:56 | CD8α hinge region |
| TTTPAPRPPTPAPTIASQPLSLRPEACRPAAGGAVHTRGLDFAC | |
| SEQ ID NO:57 | CD8α transmembrane region |
| YIWAPLAGTCGVLLLSLVITLYC | |
| SEQ ID NO:58 | 4-1BB cytoplasmic domain |
| KRGRKKLLYIFKQPFMRPVQTTQEEDGCSCRFPEEEEGGCEL | |
| SEQ ID NO:59 | CD3ζ |
| RVKFSRSADAPAYQQGQNQLYNELNLGRREEYDVLDKRRGRDPEMGGKPRRKNPQEGL YNELQKDKMAEAYSEIGMKGERRRGKGHDGLYQGLSTATKDTYDALHMQALPPR | |
| SEQ ID NO:60 | hu806 CAR amino acid sequence |
| MALPVTALLLPLALLLHAARPQVQLQESGPGLVKPSQTLSLTCTVSGYSISSDFAWNWIR | |
| QPPGKGLEWMGYISYSGNTRYQPSLKSRITISRDTSKNQFFLKLNSVTAADTATYYCVTA GRGFPYWGQGTLVTVSSGGGGSGGGGSGGGGSDIQMTQSPSSMSVSVGDRVTITCHSSQ DINSNIGWLQQKPGKSFKGLIYHGTNLDDGVPSRFSGSGSGTDYTLTISSLQPEDFATYYC VQYAQFPWTFGGGTKLEIKRTRTTTPAPRPPTPAPTIASQPLSLRPEACRPAAGGAVHTRG LDFACDIYIWAPLAGTCGVLLLSLVITLYCKRGRKKLLYIFKQPFMRPVQTTQEEDGCSCR FPEEEEGGCELRVKFSRSADAPAYQQGQNQLYNELNLGRREEYDVLDKRRGRDPEMGG KPRRKNPQEGLYNELQKDKMAEAYSEIGMKGERRRGKGHDGLYQGLSTATKDTYDALH MQALPPR* | |
| SEQ ID NO:61 | E2 CAR amino acid sequence |
| MALPVTALLLPLALLLHAARPEVQLLESGGGLVQPGGSLRLSCAASGFTFSNYDMSWVR QAPGKGLEWVSGISHSSGSKYYADSVKGRFTISRDNSKNTLYLQMNSLRAEDTAVYYCA KDATPRPLKPFDYWGQGTLVTVSSGGGGSGGGGSGGGGSQSVLTQPPSASGTPGQRVTIS CTGSSSNIGNNDVSWYQQLPGTAPKLLIYDDNKRPSGVPDRFSGSKSGTSASLAISGLRSE DEADYYCGSWDASLNAYVFGGGTKLTVLGTRTTTPAPRPPTPAPTIASQPLSLRPEACRP AAGGAVHTRGLDFACDIYIWAPLAGTCGVLLLSLVITLYCKRGRKKLLYIFKQPFMRPVQ TTQEEDGCSCRFPEEEEGGCELRVKFSRSADAPAYQQGQNQLYNELNLGRREEYDVLDK RRGRDPEMGGKPRRKNPQEGLYNELQKDKMAEAYSEIGMKGERRRGKGHDGLYQGLST ATKDTYDALHMQALPPR* | |
| SEQ ID NO:62 | Pan CAR amino acid sequence |
| MALPVTALLLPLALLLHAARPQVQLQESGPGLVKPSETLSLTCTVSGGSVSSGDYYWTWI RQSPGKGLEWIGHIYYSGNTNYNPSLKSRLTISIDTSKTQFSLKLSSVTAADTAIYYCVRDR VTGAFDIWGQGTMVTVSSASTKGPSVFPLAPCSRGGGGSGGGGSGGGGSDIQMTQSPSSL SASVGDRVTITCQASQDISNYLNWYQQKPGKAPKLLIYDASNLETGVPSRFSGSGSGTDFT FTISSLQPEDIATYFCQHFDHLPLAFGGGTKVEIKTRTTTPAPRPPTPAPTIASQPLSLRPEAC RPAAGGAVHTRGLDFACDIYIWAPLAGTCGVLLLSLVITLYCKRGRKKLLYIFKQPFMRP VQTTQEEDGCSCRFPEEEEGGCELRVKFSRSADAPAYQQGQNQLYNELNLGRREEYDVL DKRRGRDPEMGGKPRRKNPQEGLYNELQKDKMAEAYSEIGMKGERRRGKGHDGLYQG LSTATKDTYDALHMQALPPR | |
| SEQ ID NO:63 | Nec CAR amino acid sequence |
| MALPVTALLLPLALLLHAARPQVQLQESGPGLVKPSQTLSLTCTVSGGSISSGDYYWSWI RQPPGKGLEWIGYIYYSGSTDYNPSLKSRVTMSVDTSKNQFSLKVNSVTAADTAVYYCA RVSIFGVGTFDYWGQGTLVTVSSGGGGSGGGGSGGGGSEIVMTQSPATLSLSPGERATLS CRASQSVSSYLAWYQQKPGQAPRLLIYDASNRATGIPARFSGSGSGTDFTLTISSLEPEDFA VYYCHQYGSTPLTFGGGTKAEIKTRTTTPAPRPPTPAPTIASQPLSLRPEACRPAAGGAVH TRGLDFACDIYIWAPLAGTCGVLLLSLVITLYCKRGRKKLLYIFKQPFMRPVQTTQEEDGC SCRFPEEEEGGCELRVKFSRSADAPAYQQGQNQLYNELNLGRREEYDVLDKRRGRDPEM GGKPRRKNPQEGLYNELQKDKMAEAYSEIGMKGERRRGKGHDGLYQGLSTATKDTYD ALHMQALPPR | |
| SEQ ID NO:64 | Nimo CAR amino acid sequence |
| MALPVTALLLPLALLLHAARPDIQMTQSPSSLSASVGDRVTITCRSSQNIVHSNGNTYLD WYQQTPGKAPKLLIYKVSNRFSGVPSRFSGSGSGTDFTFTISSLQPEDIATYYCFQYSHVP WTFGQGTKLQITREGGGGSGGGGSGGGGSQVQLQQSGAEVKKPGSSVKVSCKASGYTFT NYYIYWVRQAPGQGLEWIGGINPTSGGSNFNEKFKTRVTITVDESTNTAYMELSSLRSED TAFYFCARQGLWFDSDGRGFDFWGQGSTVTVSSTRTTTPAPRPPTPAPTIASQPLSLRPEA CRPAAGGAVHTRGLDFACDIYIWAPLAGTCGVLLLSLVITLYCKRGRKKLLYIFKQPFMR PVQTTQEEDGCSCRFPEEEEGGCELRVKFSRSADAPAYQQGQNQLYNELNLGRREEYDV LDKRRGRDPEMGGKPRRKNPQEGLYNELQKDKMAEAYSEIGMKGERRRGKGHDGLYQ GLSTATKDTYDALHMQALPPR | |
| SEQ ID NO:65 | 301 CAR amino acid sequence |
| MALPVTALLLPLALLLHAARPMAQVQLVQSGAEVKKPGSSVKVSCKASGGTFSSYAISW VRQAPGQGLEWMGGIIPIFGTANYAQKFQGRVTITADESTSTAYMELSSLRSEDTAVYYC ARTRLKHQWGQGTLVTVSSGGGGSGGGGSGGSALSSELTQDPAVSVALGQTVRITCQGD | |
| SLRSYYASWYQQKPGQAPVLVIYGKNNRPSGIPDRFSGSSSGNTASLTITGAQAEDEADY YCNSRDSSGPVFGGGTKLTVLGAAATRTTTPAPRPPTPAPTIASQPLSLRPEACRPAAGGA VHTRGLDFACDIYIWAPLAGTCGVLLLSLVITLYCKRGRKKLLYIFKQPFMRPVQTTQEE DGCSCRFPEEEEGGCELRVKFSRSADAPAYQQGQNQLYNELNLGRREEYDVLDKRRGRD PEMGGKPRRKNPQEGLYNELQKDKMAEAYSEIGMKGERRRGKGHDGLYQGLSTATKDT YDALHMQALPPR | |
| SEQ ID NO:66 | hu806 CAR nucleotide sequence |
| ATGGCCTTACCAGTGACCGCCTTGCTCCTGCCGCTGGCCTTGCTGCTCCACGCCGCCA GGCCGCAGGTGCAGCTCCAAGAGAGTGGACCTGGGCTTGTCAAGCCGAGTCAAACTT TGTCCCTAACATGTACTGTGTCCGGATACTCTATCTCATCAGATTTTGCGTGGAATTGGA TAAGGCAGCCACCAGGGAAAGGTTTAGAATGGATGGGCTACATATCATACTCTGGGAA CACCAGATATCAACCTTCTCTGAAAAGCCGGATCACAATCTCAAGGGACACGTCGAAG AATCAGTTCTTCCTGAAACTGAACTCCGTTACAGCCGCAGACACAGCAACATATTACTG CGTAACCGCTGGCAGAGGCTTCCCCTATTGGGGACAGGGCACCCTAGTGACAGTGAGC AGCGGTGGCGGTGGCTCGGGCGGTGGTGGGTCGGGTGGCGGCGGATCTGATATTCAGA TGACTCAGAGTCCATCCAGCATGTCAGTCTCCGTGGGAGATAGGGTGACGATAACCTG TCATTCAAGCCAAGACATCAACTCCAATATTGGATGGCTCCAACAGAAGCCTGGTAAG TCCTTCAAAGGACTAATCTATCACGGAACAAACTTGGACGACGGCGTGCCATCGAGAT TTTCAGGGTCTGGCAGCGGGACCGACTATACACTGACCATCTCTAGCTTACAACCAGA GGACTTTGCCACATACTACTGCGTCCAGTACGCTCAGTTCCCCTGGACATTCGGCGGCG GCACAAAACTGGAAATCAAACGTACGCGTACCACGACGCCAGCGCCGCGACCACCAA CACCGGCGCCCACCATCGCGTCGCAGCCCCTGTCCCTGCGCCCAGAGGCGTGCCGGCC AGCGGCGGGGGGCGCAGTGCACACGAGGGGGCTGGACTTCGCCTGTGATATCTACATC TGGGCGCCCTTGGCCGGGACTTGTGGGGTCCTTCTCCTGTCACTGGTTATCACCCTTTA CTGCAAACGGGGCAGAAAGAAACTCCTGTATATATTCAAACAACCATTTATGAGACCA GTACAAACTACTCAAGAGGAAGATGGCTGTAGCTGCCGATTTCCAGAAGAAGAAGAA GGAGGATGTGAACTGAGAGTGAAGTTCAGCAGGAGCGCAGACGCCCCCGCGTACCAG CAGGGCCAGAACCAGCTCTATAACGAGCTCAATCTAGGACGAAGAGAGGAGTACGAT GTTTTGGACAAGAGACGTGGCCGGGACCCTGAGATGGGGGGAAAGCCGAGAAGGAA GAACCCTCAGGAAGGCCTGTACAATGAACTGCAGAAAGATAAGATGGCGGAGGCCTA CAGTGAGATTGGGATGAAAGGCGAGCGCCGGAGGGGCAAGGGGCACGATGGCCTTTA CCAGGGTCTCAGTACAGCCACCAAGGACACCTACGACGCCCTTCACATGCAGGCCCTG CCCCCTCGCTAA | |
| SEQ ID NO:67 | E2 CAR nucleotide sequence |
| ATGGCCTTACCAGTGACCGCCTTGCTCCTGCCGCTGGCCTTGCTGCTCCACGCCGCCA GGCCGGAGGTGCAGCTGTTGGAGTCTGGGGGAGGCTTGGTACAGCCTGGGGGGTCCC TGAGACTCTCCTGTGCAGCCTCTGGATTCACCTTTAGCAATTATGATATGAGCTGGGTC CGCCAGGCTCCAGGGAAGGGGCTGGAGTGGGTCTCAGGGATCTCTCATAGTAGTGGTA GTAAATATTACGCTGATTCTGTAAAAGGTCGGTTCACCATCTCCAGAGACAATTCCAAG AACACGCTGTATCTGCAAATGAACAGCCTGAGAGCCGAGGACACGGCCGTGTATTACT GTGCGAAAGATGCTACTCCGCGTCCGCTGAAGCCTTTCGACTACTGGGGCCAGGGTAC ACTGGTCACCGTGAGCTCAGGTGGCGGTGGCTCGGGCGGTGGTGGGTCGGGTGGCGG CGGATCTCAGTCTGTGCTGACTCAGCCACCCTCAGCGTCTGGGACCCCCGGGCAGAGG GTCACCATCTCTTGTACTGGCTCTTCATCTAATATTGGCAATAATGATGTCTCCTGGTACC AGCAGCTCCCAGGAACGGCCCCCAAACTCCTCATCTATGATGATAATAAGCGGCCAAG CGGGGTCCCTGACCGATTCTCTGGCTCCAAATCTGGCACCTCAGCCTCCCTGGCCATCA GTGGGCTCCGGTCCGAGGATGAGGCTGATTATTACTGTGGTTCTTGGGATGCTAGCCTG AATGCTTATGTCTTCGGCGGAGGCACCAAGCTGACGGTCCTAGGCACGCGTACCACGA CGCCAGCGCCGCGACCACCAACACCGGCGCCCACCATCGCGTCGCAGCCCCTGTCCCT GCGCCCAGAGGCGTGCCGGCCAGCGGCGGGGGGCGCAGTGCACACGAGGGGGCTGG ACTTCGCCTGTGATATCTACATCTGGGCGCCCTTGGCCGGGACTTGTGGGGTCCTTCTC CTGTCACTGGTTATCACCCTTTACTGCAAACGGGGCAGAAAGAAACTCCTGTATATATT CAAACAACCATTTATGAGACCAGTACAAACTACTCAAGAGGAAGATGGCTGTAGCTGC CGATTTCCAGAAGAAGAAGAAGGAGGATGTGAACTGAGAGTGAAGTTCAGCAGGAG CGCAGACGCCCCCGCGTACCAGCAGGGCCAGAACCAGCTCTATAACGAGCTCAATCTA | |
| GGACGAAGAGAGGAGTACGATGTTTTGGACAAGAGACGTGGCCGGGACCCTGAGATG GGGGGAAAGCCGAGAAGGAAGAACCCTCAGGAAGGCCTGTACAATGAACTGCAGAA AGATAAGATGGCGGAGGCCTACAGTGAGATTGGGATGAAAGGCGAGCGCCGGAGGGG CAAGGGGCACGATGGCCTTTACCAGGGTCTCAGTACAGCCACCAAGGACACCTACGA CGCCCTTCACATGCAGGCCCTGCCCCCTCGCTAA | |
| SEQ ID NO:68 | Pan CAR nucleotide sequence |
| ATGGCCTTACCAGTGACCGCCTTGCTCCTGCCGCTGGCCTTGCTGCTCCACGCCGCCA GGCCGCAGGTGCAACTTCAAGAGAGCGGCCCTGGACTTGTGAAACCCAGCGAAACCC TGAGCCTGACATGTACCGTGAGCGGAGGAAGCGTGAGCAGCGGAGACTACTACTGGA CCTGGATCAGACAGAGCCCCGGAAAAGGCCTTGAATGGATCGGCCACATCTACTACAG CGGCAACACCAACTACAACCCCAGCCTGAAGAGCAGACTGACCATCAGCATCGACAC CAGCAAGACCCAGTTCAGCCTGAAGCTGAGCAGCGTGACAGCTGCTGACACCGCTAT CTACTACTGCGTGAGAGACAGAGTGACCGGCGCTTTCGATATTTGGGGCCAGGGAACA ATGGTGACCGTGAGCAGCGCTAGCACAAAAGGCCCTAGCGTGTTTCCTCTTGCCCCCT GTAGCAGAGGTGGCGGTGGCTCGGGCGGTGGTGGGTCGGGTGGCGGCGGATCTGACA TCCAGATGACACAGAGCCCTAGCAGCCTGTCTGCTAGCGTTGGAGACAGAGTGACCAT CACCTGTCAAGCCAGCCAGGACATCAGCAACTACCTGAACTGGTACCAGCAGAAACC TGGCAAGGCTCCCAAACTGCTGATTTACGACGCCAGCAATCTGGAAACAGGCGTGCCT AGCAGATTTAGCGGCAGCGGAAGCGGAACCGATTTCACCTTCACCATCAGCAGCCTGC AGCCCGAAGACATTGCCACCTACTTCTGCCAGCACTTCGATCATCTGCCCCTGGCCTTT GGAGGAGGAACAAAGGTGGAGATCAAGACGCGTACCACGACGCCAGCGCCGCGACC ACCAACACCGGCGCCCACCATCGCGTCGCAGCCCCTGTCCCTGCGCCCAGAGGCGTG CCGGCCAGCGGCGGGGGGCGCAGTGCACACGAGGGGGCTGGACTTCGCCTGTGATAT CTACATCTGGGCGCCCTTGGCCGGGACTTGTGGGGTCCTTCTCCTGTCACTGGTTATCA CCCTTTACTGCAAACGGGGCAGAAAGAAACTCCTGTATATATTCAAACAACCATTTATG AGACCAGTACAAACTACTCAAGAGGAAGATGGCTGTAGCTGCCGATTTCCAGAAGAA GAAGAAGGAGGATGTGAACTGAGAGTGAAGTTCAGCAGGAGCGCAGACGCCCCCGC GTACCAGCAGGGCCAGAACCAGCTCTATAACGAGCTCAATCTAGGACGAAGAGAGGA GTACGATGTTTTGGACAAGAGACGTGGCCGGGACCCTGAGATGGGGGGAAAGCCGAG AAGGAAGAACCCTCAGGAAGGCCTGTACAATGAACTGCAGAAAGATAAGATGGCGGA GGCCTACAGTGAGATTGGGATGAAAGGCGAGCGCCGGAGGGGCAAGGGGCACGATGG CCTTTACCAGGGTCTCAGTACAGCCACCAAGGACACCTACGACGCCCTTCACATGCAG GCCCTGCCCCCTCGCTAA | |
| SEQ ID NO:69 | Nec CAR nucleotide sequence |
| ATGGCCTTACCAGTGACCGCCTTGCTCCTGCCGCTGGCCTTGCTGCTCCACGCCGCCA GGCCGCAGGTGCAGCTGCAGGAGTCGGGCCCAGGACTGGTGAAGCCTTCACAGACCC TGTCCCTCACCTGCACTGTCTCTGGTGGCTCCATCAGCAGTGGTGATTACTACTGGAGT TGGATCCGCCAGCCCCCAGGGAAGGGCCTGGAGTGGATTGGGTACATCTATTACAGTG GGAGCACCGACTACAACCCGTCCCTCAAGAGTCGAGTCACCATGTCCGTAGACACGTC CAAGAATCAGTTTTCCCTGAAGGTCAACTCTGTGACCGCCGCAGACACGGCTGTGTAT TACTGTGCGAGAGTGTCGATTTTTGGAGTGGGGACATTTGACTACTGGGGCCAGGGCA CCCTGGTCACCGTCTCAAGCGGTGGCGGTGGCTCGGGCGGTGGTGGGTCGGGTGGCG GCGGATCTGAAATTGTGATGACACAGTCTCCAGCCACCCTGTCTTTGTCTCCAGGGGA AAGAGCCACCCTCTCCTGCAGGGCCAGTCAGAGTGTTAGCAGCTACTTAGCCTGGTAC CAACAGAAACCTGGCCAGGCTCCCAGGCTCCTCATCTATGATGCATCCAACAGGGCCA CTGGCATCCCAGCCAGGTTCAGTGGCAGTGGGTCTGGGACAGACTTCACTCTCACCAT CAGCAGCCTAGAGCCTGAAGATTTTGCAGTGTATTACTGTCACCAGTATGGTAGCACAC CTCTCACTTTCGGCGGAGGGACCAAGGCGGAGATCAAAACGCGTACCACGACGCCAG CGCCGCGACCACCAACACCGGCGCCCACCATCGCGTCGCAGCCCCTGTCCCTGCGCCC AGAGGCGTGCCGGCCAGCGGCGGGGGGCGCAGTGCACACGAGGGGGCTGGACTTCG CCTGTGATATCTACATCTGGGCGCCCTTGGCCGGGACTTGTGGGGTCCTTCTCCTGTCA CTGGTTATCACCCTTTACTGCAAACGGGGCAGAAAGAAACTCCTGTATATATTCAAACA ACCATTTATGAGACCAGTACAAACTACTCAAGAGGAAGATGGCTGTAGCTGCCGATTT CCAGAAGAAGAAGAAGGAGGATGTGAACTGAGAGTGAAGTTCAGCAGGAGCGCAGA CGCCCCCGCGTACCAGCAGGGCCAGAACCAGCTCTATAACGAGCTCAATCTAGGACGA | |
| AGAGAGGAGTACGATGTTTTGGACAAGAGACGTGGCCGGGACCCTGAGATGGGGGGA AAGCCGAGAAGGAAGAACCCTCAGGAAGGCCTGTACAATGAACTGCAGAAAGATAAG ATGGCGGAGGCCTACAGTGAGATTGGGATGAAAGGCGAGCGCCGGAGGGGCAAGGG GCACGATGGCCTTTACCAGGGTCTCAGTACAGCCACCAAGGACACCTACGACGCCCTT CACATGCAGGCCCTGCCCCCTCGCTAA | |
| SEQ ID NO:70 | Nimo CAR nucleotide sequence |
| ATGGCCTTACCAGTGACCGCCTTGCTCCTGCCGCTGGCCTTGCTGCTCCACGCCGCCA GGCCGGACATCCAGATGACCCAGAGCCCAAGCAGCCTGAGCGCCAGCGTGGGTGACA GAGTGACCATCACCTGTAGATCTAGTCAGAACATTGTACATAGTAATGGAAACACCTAT TTAGACTGGTACCAGCAGACGCCAGGTAAGGCTCCAAAGCTGCTGATCTACAAAGTTT CCAACCGATTTAGCGGTGTGCCAAGCAGATTCAGCGGTAGCGGTAGCGGTACCGACTT CACCTTCACCATCAGCAGCCTCCAGCCAGAGGACATCGCCACCTACTACTGCTTTCAAT ACTCACATGTTCCGTGGACGTTTGGTCAGGGTACTAAGCTTCAAATCACTAGGGAAGG TGGCGGTGGCTCGGGCGGTGGTGGGTCGGGTGGCGGCGGATCTCAGGTCCAACTGCA GCAGTCTGGGGCTGAAGTGAAGAAGCCTGGGTCATCAGTGAAGGTGTCCTGCAAGGC TTCTGGCTACACCTTCACCAACTACTATATTTACTGGGTGCGACAGGCCCCTGGACAAG GGCTTGAGTGGATTGGAGGGATTAATCCTACTAGCGGTGGTAGCAACTTCAATGAGAA GTTCAAGACTAGAGTGACCATCACCGTGGACGAATCCACGAACACAGCCTACATGGAG CTGAGCAGCCTGAGATCTGAGGACACGGCCTTCTATTTCTGTGCGAGACAAGGGCTTT GGTTTGATAGCGATGGGCGAGGTTTTGATTTCTGGGGCCAAGGGTCTACGGTCACCGT CTCCTCAACGCGTACCACGACGCCAGCGCCGCGACCACCAACACCGGCGCCCACCAT CGCGTCGCAGCCCCTGTCCCTGCGCCCAGAGGCGTGCCGGCCAGCGGCGGGGGGCGC AGTGCACACGAGGGGGCTGGACTTCGCCTGTGATATCTACATCTGGGCGCCCTTGGCC GGGACTTGTGGGGTCCTTCTCCTGTCACTGGTTATCACCCTTTACTGCAAACGGGGCA GAAAGAAACTCCTGTATATATTCAAACAACCATTTATGAGACCAGTACAAACTACTCAA GAGGAAGATGGCTGTAGCTGCCGATTTCCAGAAGAAGAAGAAGGAGGATGTGAACTG AGAGTGAAGTTCAGCAGGAGCGCAGACGCCCCCGCGTACCAGCAGGGCCAGAACCA GCTCTATAACGAGCTCAATCTAGGACGAAGAGAGGAGTACGATGTTTTGGACAAGAGA CGTGGCCGGGACCCTGAGATGGGGGGAAAGCCGAGAAGGAAGAACCCTCAGGAAGG CCTGTACAATGAACTGCAGAAAGATAAGATGGCGGAGGCCTACAGTGAGATTGGGATG AAAGGCGAGCGCCGGAGGGGCAAGGGGCACGATGGCCTTTACCAGGGTCTCAGTACA GCCACCAAGGACACCTACGACGCCCTTCACATGCAGGCCCTGCCCCCTCGCTAA | |
| SEQ ID NO:71 | 301 CAR nucleotide sequence |
| ATGGCCTTACCAGTGACCGCCTTGCTCCTGCCGCTGGCCTTGCTGCTCCACGCCGCCA GGCCGATGGCCCAGGTGCAGCTGGTGCAGTCTGGGGCTGAGGTGAAGAAGCCTGGGT CCTCGGTGAAGGTCTCCTGCAAGGCTTCTGGAGGCACCTTCAGCAGCTATGCTATCAG CTGGGTGCGACAGGCCCCTGGACAAGGGCTTGAGTGGATGGGAGGGATCATCCCTATC TTTGGTACAGCAAACTACGCACAGAAGTTCCAGGGCAGAGTCACGATTACCGCGGAC GAATCCACGAGCACAGCCTACATGGAGCTGAGCAGCCTGAGATCTGAGGACACGGCC GTGTATTACTGTGCAAGAACTCGGCTTAAGCATCAGTGGGGCCAAGGTACCCTGGTCA CCGTCTCGAGTGGTGGAGGCGGTTCAGGCGGAGGTGGCTCTGGCGGTAGTGCACTTTC TTCTGAGCTGACTCAGGACCCTGCTGTGTCTGTGGCCTTGGGACAGACAGTCAGGATC ACATGCCAAGGAGACAGCCTCAGAAGCTATTATGCAAGCTGGTACCAGCAGAAGCCA GGACAGGCCCCTGTACTTGTCATCTATGGTAAAAACAACCGGCCCTCAGGGATCCCAG ACCGATTCTCTGGCTCCAGCTCAGGAAACACAGCTTCCTTGACCATCACTGGGGCTCA GGCGGAAGATGAGGCTGACTATTACTGTAACTCCCGGGACAGCAGTGGTCCGGTATTC GGCGGAGGGACCAAGCTGACCGTCCTAGGTGCGGCCGCAACGCGTACCACGACGCCA GCGCCGCGACCACCAACACCGGCGCCCACCATCGCGTCGCAGCCCCTGTCCCTGCGCC CAGAGGCGTGCCGGCCAGCGGCGGGGGGCGCAGTGCACACGAGGGGGCTGGACTTC GCCTGTGATATCTACATCTGGGCGCCCTTGGCCGGGACTTGTGGGGTCCTTCTCCTGTC ACTGGTTATCACCCTTTACTGCAAACGGGGCAGAAAGAAACTCCTGTATATATTCAAAC AACCATTTATGAGACCAGTACAAACTACTCAAGAGGAAGATGGCTGTAGCTGCCGATT TCCAGAAGAAGAAGAAGGAGGATGTGAACTGAGAGTGAAGTTCAGCAGGAGCGCAG ACGCCCCCGCGTACCAGCAGGGCCAGAACCAGCTCTATAACGAGCTCAATCTAGGACG AAGAGAGGAGTACGATGTTTTGGACAAGAGACGTGGCCGGGACCCTGAGATGGGGGG | |
| AAAGCCGAGAAGGAAGAACCCTCAGGAAGGCCTGTACAATGAACTGCAGAAAGATAA GATGGCGGAGGCCTACAGTGAGATTGGGATGAAAGGCGAGCGCCGGAGGGGCAAGG GGCACGATGGCCTTTACCAGGGTCTCAGTACAGCCACCAAGGACACCTACGACGCCCT TCACATGCAGGCCCTGCCCCCTCGCTAA | |
| SEQ ID NO:72 | Peptide linker |
| GGGGSGGGGSGGGGS | |
| SEQ ID NO:73 m806 ScFv | |
| DVQLQESGPSLVKPSQSLSLTCTVTGYSITSDFAWNWIRQFPGNKLEWMGYISYSGNTRYN PSLKSRISITRDTSKNQFFLQLNSVTIEDTATYYCVTAGRGFPYWGQGTLVTVSAGGGGSG GGGSGGGGSDILMTQSPSSMSVSLGDTVSITCHSSQDINSNIGWLQQRPGKSFKGLIYHGT NLDDEVPSRFSGSGSGADYSLTISSLESEDFADYYCVQYAQFPWTFGGGTKLEIKR | |
| SEQ ID NO:74 m806 ScFv nucleotide sequence | |
| GATGTGCAGCTTCAGGAGTCGGGACCTAGCCTGGTGAAACCTTCTCAGTCTCTGTCCC TCACCTGCACTGTCACTGGCTACTCAATCACCAGTGATTTTGCCTGGAACTGGATCCGG CAGTTTCCAGGAAACAAGCTGGAGTGGATGGGCTACATAAGTTATAGTGGTAACACTA GGTACAACCCATCTCTCAAAAGTCGAATCTCTATCACTCGAGACACATCCAAGAACCA ATTCTTCCTGCAGTTGAATTCTGTGACTATTGAGGACACAGCCACATATTACTGTGTAAC GGCGGGACGCGGGTTTCCTTATTGGGGCCAAGGGACTCTGGTCACTGTCTCTGCAGGT GGCGGTGGCTCGGGCGGTGGTGGGTCGGGTGGCGGCGGATCTGACATCCTGATGACC CAATCTCCATCCTCCATGTCTGTATCTCTGGGAGACACAGTCAGCATCACTTGCCATTC AAGTCAGGACATTAACAGTAATATAGGGTGGTTGCAGCAGAGACCAGGGAAATCATTT AAGGGCCTGATCTATCATGGAACCAACTTGGACGATGAAGTTCCATCAAGGTTCAGTG GCAGTGGATCTGGAGCCGATTATTCTCTCACCATCAGCAGCCTGGAATCTGAAGATTTT GCAGACTATTACTGTGTACAGTATGCTCAGTTTCCGTGGACGTTCGGTGGAGGCACCA AGCTGGAAATCAAACGT | |
| SEQ ID NO:75 m806 ScFv VH CDR1 | |
| SDFAWN | |
| SEQ ID NO:76 m806 ScFv VH CDR2 | |
| YISYSGNTRYNPSLKS | |
| SEQ ID NO:77 m806 ScFv VH CDR3 | |
| VTAGRGFPY | |
| SEQ ID NO:78 m806 ScFv VL CDR1 | |
| HSSQDINSNIG | |
| SEQ ID NO:79 m806 ScFv VL CDR2 | |
| HGTNLDD | |
| SEQ ID NO:80 m806 ScFv VL CDR3 | |
| VQYAQFPWT | |
| SEQ ID NO:81 m806 CAR | |
| MALPVTALLLPLALLLHAARPDVQLQESGPSLVKPSQSLSLTCTVTGYSITSDFAWNWIRQ FPGNKLEWMGYISYSGNTRYNPSLKSRISITRDTSKNQFFLQLNSVTIEDTATYYCVTAGR GFPYWGQGTLVTVSAGGGGSGGGGSGGGGSDILMTQSPSSMSVSLGDTVSITCHSSQDIN SNIGWLQQRPGKSFKGLIYHGTNLDDEVPSRFSGSGSGADYSLTISSLESEDFADYYCVQY AQFPWTFGGGTKLEIKRTRTTTPAPRPPTPAPTIASQPLSLRPEACRPAAGGAVHTRGLDFA CDIYIWAPLAGTCGVLLLSLVITLYCKRGRKKLLYIFKQPFMRPVQTTQEEDGCSCRFPEEE EGGCELRVKFSRSADAPAYQQGQNQLYNELNLGRREEYDVLDKRRGRDPEMGGKPRRK NPQEGLYNELQKDKMAEAYSEIGMKGERRRGKGHDGLYQGLSTATKDTYDALHMQALP PR* | |
| SEQ ID NO:82 m806 CAR nucleotide sequence | |
| GGATCCATGGCCTTACCAGTGACCGCCTTGCTCCTGCCGCTGGCCTTGCTGCTCCACGC CGCCAGGCCGGATGTGCAGCTTCAGGAGTCGGGACCTAGCCTGGTGAAACCTTCTCAG TCTCTGTCCCTCACCTGCACTGTCACTGGCTACTCAATCACCAGTGATTTTGCCTGGAA CTGGATCCGGCAGTTTCCAGGAAACAAGCTGGAGTGGATGGGCTACATAAGTTATAGT GGTAACACTAGGTACAACCCATCTCTCAAAAGTCGAATCTCTATCACTCGAGACACATC CAAGAACCAATTCTTCCTGCAGTTGAATTCTGTGACTATTGAGGACACAGCCACATATT ACTGTGTAACGGCGGGACGCGGGTTTCCTTATTGGGGCCAAGGGACTCTGGTCACTGT CTCTGCAGGTGGCGGTGGCTCGGGCGGTGGTGGGTCGGGTGGCGGCGGATCTGACAT CCTGATGACCCAATCTCCATCCTCCATGTCTGTATCTCTGGGAGACACAGTCAGCATCA CTTGCCATTCAAGTCAGGACATTAACAGTAATATAGGGTGGTTGCAGCAGAGACCAGG GAAATCATTTAAGGGCCTGATCTATCATGGAACCAACTTGGACGATGAAGTTCCATCAA GGTTCAGTGGCAGTGGATCTGGAGCCGATTATTCTCTCACCATCAGCAGCCTGGAATCT GAAGATTTTGCAGACTATTACTGTGTACAGTATGCTCAGTTTCCGTGGACGTTCGGTGG AGGCACCAAGCTGGAAATCAAACGTACGCGTACCACGACGCCAGCGCCGCGACCACC AACACCGGCGCCCACCATCGCGTCGCAGCCCCTGTCCCTGCGCCCAGAGGCGTGCCG GCCAGCGGCGGGGGGCGCAGTGCACACGAGGGGGCTGGACTTCGCCTGTGATATCTA CATCTGGGCGCCCTTGGCCGGGACTTGTGGGGTCCTTCTCCTGTCACTGGTTATCACCC TTTACTGCAAACGGGGCAGAAAGAAACTCCTGTATATATTCAAACAACCATTTATGAGA CCAGTACAAACTACTCAAGAGGAAGATGGCTGTAGCTGCCGATTTCCAGAAGAAGAA GAAGGAGGATGTGAACTGAGAGTGAAGTTCAGCAGGAGCGCAGACGCCCCCGCGTAC CAGCAGGGCCAGAACCAGCTCTATAACGAGCTCAATCTAGGACGAAGAGAGGAGTAC GATGTTTTGGACAAGAGACGTGGCCGGGACCCTGAGATGGGGGGAAAGCCGAGAAG GAAGAACCCTCAGGAAGGCCTGTACAATGAACTGCAGAAAGATAAGATGGCGGAGGC CTACAGTGAGATTGGGATGAAAGGCGAGCGCCGGAGGGGCAAGGGGCACGATGGCCT TTACCAGGGTCTCAGTACAGCCACCAAGGACACCTACGACGCCCTTCACATGCAGGCC CTGCCCCCTCGCTAAGTCGAC | |

## Claims

1. A chimeric antigen receptor (CAR) targeting EGFR, comprising an extracellular antigen binding domain specifically targeting EGFR, wherein the extracellular antigen binding domain comprises a heavy chain variable region (VH) and a light chain variable region (VL), wherein
i) the VH comprises VH-CDR1 shown in SEQ ID NO: 1, VH-CDR2 shown in SEQ ID NO: 2, and VH-CDR3 shown in SEQ ID NO: 3, and the VL comprises VL-CDR1 shown in SEQ ID NO: 4, VL-CDR2 shown in SEQ ID NO: 5, and VL-CDR3 shown in SEQ ID NO: 6;
ii) the VH comprises VH-CDR1 shown in SEQ ID NO: 10, VH-CDR2 shown in SEQ ID NO: 11, and VH-CDR3 shown in SEQ ID NO: 12, and the VL comprises VL-CDR1 shown in SEQ ID NO: 13, VL-CDR2 shown in SEQ ID NO: 14, and VL-CDR3 shown in SEQ ID NO: 16;
iii) the VH comprises VH-CDR1 shown in SEQ ID NO: 19, VH-CDR2 shown in SEQ ID NO: 20, and VH-CDR3 shown in SEQ ID NO: 21, and the VL comprises VL-CDR1 shown in SEQ ID NO: 22, VL-CDR2 shown in SEQ ID NO: 23, and VL-CDR3 shown in SEQ ID NO: 24;
iv) the VH comprises VH-CDR1 shown in SEQ ID NO: 28, VH-CDR2 shown in SEQ ID NO: 29, and VH-CDR3 shown in SEQ ID NO: 30, and the VL comprises VL-CDR1 shown in SEQ ID NO: 31, VL-CDR2 shown in SEQ ID NO: 32, and VL-CDR3 shown in SEQ ID NO: 33;
v) the VH comprises VH-CDR1 shown in SEQ ID NO: 37, VH-CDR2 shown in SEQ ID NO: 38, and VH-CDR3 shown in SEQ ID NO: 39, and the VL comprises VL-CDR1 shown in SEQ ID NO: 40, VL-CDR2 shown in SEQ ID NO: 41, and VL-CDR3 shown in SEQ ID NO: 42; or
vi) the VH comprises VH-CDR1 shown in SEQ ID NO: 46, VH-CDR2 shown in SEQ ID NO: 47, and VH-CDR3 shown in SEQ ID NO: 48, and the VL comprises VL-CDR1 shown in SEQ ID NO: 49, VL-CDR2 shown in SEQ ID NO: 50, and VL-CDR3 shown in SEQ ID NO: 51.

2. The CAR targeting EGFR according to claim 1, wherein
i) the VH comprises the amino acid sequence shown in SEQ ID NO: 7, and the VL comprises the amino acid sequence shown in SEQ ID NO: 8;
ii) the VH comprises the amino acid sequence shown in SEQ ID NO: 16, and the VL comprises the amino acid sequence shown in SEQ ID NO: 17;
iii) the VH comprises the amino acid sequence shown in SEQ ID NO: 25, and the VL comprises the amino acid sequence shown in SEQ ID NO: 26;
iv) the VH comprises the amino acid sequence shown in SEQ ID NO: 34, and the VL comprises the amino acid sequence shown in SEQ ID NO: 35;
v) the VH comprises the amino acid sequence shown in SEQ ID NO: 43, and the VL comprises the amino acid sequence shown in SEQ ID NO: 44; or
vi) the VH comprises the amino acid sequence shown in SEQ ID NO: 52, and the VL comprises the amino acid sequence shown in SEQ ID NO: 53.

3. The CAR targeting EGFR according to claim 1 or 2, wherein the extracellular antigen binding domain comprises a single stranded Fv fragment (scFv).

4. The CAR targeting EGFR according to claim 3, wherein the scFv comprises an amino acid sequence selected from SEQ ID NOs: 9, 18, 27, 36, 45 and 54.

5. The CAR targeting EGFR according to any one of claims 1-4, wherein the CAR further comprises a CD8α signal peptide at the N-terminus, for example, the CD8α signal peptide comprises the amino acid sequence of SEQ ID NO: 55.

6. The CAR targeting EGFR according to any one of claims 1-5, wherein the CAR further comprises a transmembrane domain, such as a CD8α transmembrane domain, for example, the CD8α transmembrane domain comprises the amino acid sequence of SEQ ID NO: 57.

7. The CAR targeting EGFR according to any one of claims 1-6, wherein the CAR further comprises a hinge region located between the extracellular antigen binding domain and the transmembrane domain, for example, the hinge region is a CD8α hinge region, for example, the CD8α hinge region comprises the amino acid sequence of SEQ ID NO: 56.

8. The CAR targeting EGFR according to any one of claims 1-7, wherein the CAR further comprises a signal transduction domain, such as a CD3ζ signal transduction domain, for example, the CD3ζ signal transduction domain comprises the amino acid sequence shown in SEQ ID NO: 59.

9. The CAR targeting EGFR according to any one of claims 1-8, wherein the CAR further comprises one or more co-stimulatory domains, such as a 4-1BB co-stimulatory domain, for example, the 4-1BB co-stimulatory domain comprises the amino acid sequence of SEQ ID NO: 58.

10. The CAR targeting EGFR according to any one of claims 1-9, wherein the CAR comprises an amino acid sequence selected from SEQ ID NOs: 60-65.

11. A therapeutic T cell, which comprises the CAR according to any one of claims 1-10.

12. The therapeutic T cell according to claim 11, wherein a TGFβ receptor in the therapeutic T cell is knocked down or knocked out.

13. The therapeutic T cell according to claim 11 or 12, wherein the therapeutic T cell can specifically lyse a tumor cell expressing EGFR in vitro at an effect-target ratio of about 0.2:1 to about 0.00625:1, for example, about 0.2:1, about 0.1:1, about 0.05:1, about 0.025:1, about 0.0125:1, and about 0.00625:1.

14. Use of the therapeutic T cell according to any one of claims 11-13 in preparation of a drug for treating an EGFR related cancer.

15. A pharmaceutical composition for treating an EGFR related cancer in a subject, which comprises a therapeutically effective amount of the therapeutic T cells according to any one of claims 11-13, and a pharmaceutically acceptable carrier.

16. A method for treating an EGFR related cancer, comprising administering a therapeutically effective amount of the therapeutic T cells according to any one of claims 11-13 or the pharmaceutical composition according to claim 15 to a subject in need.

17. The method according to claim 16, wherein the method further comprises administering a radiation therapy and/or a chemotherapy and/or another tumor targeted drug and/or an immunotherapy to the subject.

18. The use according to claim 14, the pharmaceutical composition according to claim 15 or the method according to any one of claims 16-17, wherein the EGFR related cancer is selected from esophageal cancer, gastric cancer, colon cancer, rectal cancer, colorectal cancer, pancreatic cancer, lung cancer (comprising non-small cell lung cancer NSCLC), breast cancer, cervical cancer, corpus cancer, endometrial cancer, ovarian cancer, bladder cancer, head and neck cancer (comprising head and neck squamous cell cancer SCCHN), osteosarcoma, prostate cancer, neuroblastoma, renal cancer, glioma, glioblastoma and skin cancer (comprising epithelial cancer).

19. A polynucleotide, which comprises a nucleotide sequence encoding the CAR according to any one of claims 1-10.

20. The polynucleotide according to claim 19, which comprises a nucleotide sequence selected from SEQ ID NOs: 66-71.

21. An expression construct, which comprises the polynucleotide according to claim 19 or 20 operably linked to a regulatory sequence.

22. A method for preparing the therapeutic T cell according to any one of claims 11-13, wherein the method comprises the following steps:
a) providing an isolated T cell; and
b) introducing the polynucleotide according to claim 19 or 20 or the expression construct according to claim 21 into the T cell, thereby causing the T cell to express the CAR according to any one of claims 1-10.

23. The method according to claim 22, wherein the method further comprises a step: x) knocking down or knocking out a TGFP receptor in the T cell.

24. The method according to claim 22, wherein the method further comprises a step: y) amplifying the T cell.

25. A kit for preparing the therapeutic T cell according to any one of claims 11-13.
